(19) **Europäisches Patentamt / European Patent Office / Office européen des brevets**

(11) **EP 4 782 420 A1**

(12) # EUROPEAN PATENT APPLICATION
published in accordance with Art. 153(4) EPC

(43) Date of publication:
**29.07.2026 Bulletin 2026/31**

(21) Application number: **24868380.7**

(22) Date of filing: **20.09.2024**

(51) International Patent Classification (IPC):
**C04B 35/488** (2006.01)    **A61C 5/77** (2017.01)
**A61C 13/083** (2006.01)    **A61K 6/818** (2020.01)
**A61K 6/822** (2020.01)

(52) Cooperative Patent Classification (CPC):
**A61C 5/77; A61C 13/083; A61K 6/818;
A61K 6/822; C04B 35/488**

(86) International application number:
**PCT/JP2024/033773**

(87) International publication number:
**WO 2025/063304 (27.03.2025 Gazette 2025/13)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.09.2023 JP 2023159082**

(71) Applicant: **Kuraray Noritake Dental Inc.
Kurashiki-shi
Okayama 710-0801 (JP)**

(72) Inventors:
• **KAWAI, Makito**
 **Miyoshi-shi, Aichi 470-0293 (JP)**
• **NAKANO, Kirihiro**
 **Miyoshi-shi, Aichi 470-0293 (JP)**
• **NISHIMURA, Takumi**
 **Miyoshi-shi, Aichi 470-0293 (JP)**
• **YAMAMOTO, Takahisa**
 **Nagoya-shi, Aichi 464-8601 (JP)**

(74) Representative: **D Young & Co LLP
3 Noble Street
London EC2V 7BQ (GB)**

(54) **ZIRCONIA CALCINED BODY AND METHOD FOR PRODUCING SAME**

(57)    The present invention provides a zirconia pre-sintered body that can yield a zirconia sintered body with excellent translucency even with a hold time of 2 minutes at the highest sintering temperature, and a method for producing such a zirconia pre-sintered body. The present invention relates to a zirconia pre-sintered body comprising zirconia and yttria components, along with $Y_4Zr_3O_{12}$ as a crystal system. In the zirconia pre-sintered body, the abundance $f_d$ (%) of $Y_4Zr_3O_{12}$ is preferably 2.5% or more as calculated by formula (1-1),

$$f_d\ (\%) = I_d\ /\ (I_m + I_t + I_c + I_y + I_d) \times 100 \qquad (1\text{-}1)$$

(Description of the symbols in the formula is omitted.)

EP 4 782 420 A1

**Description**

TECHNICAL FIELD

The present invention relates to a zirconia pre-sintered body, and a method for producing same. More specifically, the present invention relates to a zirconia pre-sintered body that can yield a zirconia sintered body with excellent translucency even with a hold time of 2 minutes at the highest sintering temperature, and to a method for producing such a zirconia pre-sintered body.

BACKGROUND ART

Zirconia sintered bodies find widespread applications in industry. In particular, zirconia sintered bodies have recently been used in dental material applications such as dental prostheses. In many cases, such dental prostheses are produced from a zirconia molded body of a desired shape, such as a disc or a prism, prepared by, for example, press molding zirconia particles or molding a composition containing zirconia particles. The zirconia molded body is then pre-sintered to prepare a pre-sintered body (mill blank), which is subsequently fired after being cut (milled) into a shape of the desired dental prosthesis.

Zirconia is a compound that undergoes a phase transformation between crystal systems. In this connection, partially-stabilized zirconia (PSZ) and fully-stabilized zirconia (FSZ), which exist as solid solutions of zirconia with stabilizers such as yttria (yttrium oxide; $Y_2O_3$) to prevent such phase transformations, are used in a wide variety of fields.

In the field of dentistry, zirconia material has been used as a material for frameworks because zirconia material, while possessing high strength, exhibits low translucency. In recent years, however, improvements in the translucency of zirconia material have led to increased production of zirconia-only dental prostheses.

Traditionally, the fabrication of zirconia-only dental prostheses is largely carried out in dental laboratories. This has prevented patients from receiving treatment using such dental prostheses on the day of their examination at the dental clinic, necessitating another visit on a later date for the procedure.

Alternatively, proposals have been made for one-visit treatments, where dental prostheses are conveniently fabricated at the dental clinic, enabling patients to undergo dental prosthetic treatment in a single visit. In this case, zirconia needs to be fired in a short time period, as addressed in Patent Literature 1 for example.

CITATION LIST

Patent Literature

Patent Literature 1: WO2018/056330

SUMMARY OF INVENTION

Technical Problem

The technique described in Patent Literature 1 indicates that firing a pre-sintered body for 15 minutes at a highest sintering temperature of 1,550°C can yield a sintered body that exhibits the same level of translucency as that achieved by 120-minute firing at the same temperature, suggesting that the firing time can be reduced.

However, the present inventors have found that translucency decreases when the hold time at a highest sintering temperature of 1,550°C is reduced further (for example, 7 minutes or less) in the invention according to Patent Literature 1, revealing that there remain areas for improvement with respect to further reduction of firing time in the invention according to Patent Literature 1.

It is an object of the present invention to provide a zirconia pre-sintered body that can yield a zirconia sintered body with excellent translucency even with a hold time of 2 minutes at the highest sintering temperature, and a method for producing such a zirconia pre-sintered body.

Another object of the present invention is to provide a zirconia sintered body that exhibits excellent translucency even when produced through a method that fires a zirconia pre-sintered body with a hold time of 2 minutes at the highest sintering temperature.

Solution to Problem

The present inventors conducted extensive research to find a solution to the foregoing issues, and found that a zirconia sintered body with excellent translucency can be obtained from a zirconia pre-sintered body that comprises zirconia and yttria, along with $Y_4Zr_3O_{12}$ as a crystal system, even when the hold time at the highest sintering temperature is 2 minutes. Building upon this finding, the present inventors conducted additional examinations, ultimately resulting in the completion of the present invention.

The present invention includes the following.

[1] A zirconia pre-sintered body comprising zirconia and yttria, along with $Y_4Zr_3O_{12}$ as a crystal system.

[2] The zirconia pre-sintered body according to [1], wherein the abundance $f_d$ (%) of $Y_4Zr_3O_{12}$ is 2.5% or more as calculated by the following formula (1-1),

$$f_d\ (\%) = I_d\ /\ (I_m + I_t + I_c + I_y + I_d) \times 100 \qquad (1\text{-}1)$$

wherein $f_d$ represents the abundance (%) of $Y_4Zr_3O_{12}$, $I_m$ represents the integrated intensity of the peak near $2\theta = 28.2°$, where the peak top of the main peak of the monoclinic zirconia appears in XRD measurement, $I_t$ represents the integrated intensity of the peak near $2\theta = 30.3°$, where the peak top of the main peak of the tetragonal zirconia appears in XRD measurement, $I_c$ represents the integrated intensity of the peak near $2\theta = 30.0°$, where the peak top of the main peak of the cubic zirconia appears in XRD measurement, $I_d$ represents the integrated intensity of the peak near $2\theta = 29.6°$, where the peak top of the main peak of $Y_4Zr_3O_{12}$ appears in XRD measurement, and $I_y$ represents the integrated intensity of the peak near $2\theta = 29.2°$, where the peak top of the main peak of undissolved yttria appears in XRD measurement, and

given that the main peaks of $Y_4Zr_3O_{12}$, the tetragonal zirconia, and the cubic zirconia occur at similar positions, the integrated intensity of each main peak is calculated through peak separation.

[3] The zirconia pre-sintered body according to [1] or [2], wherein the zirconia comprises monoclinic zirconia, and the monoclinic fraction $f_m$ (%) is less than 55% as calculated by the following formula (1-2),

$$f_m\ (\%) = I_m\ /\ (I_m + I_t + I_c + I_y + I_d) \times 100 \qquad (1\text{-}2),$$

wherein $f_m$ represents the monoclinic fraction (%), $I_m$ represents the integrated intensity of the peak near $2\theta = 28.2°$, where the peak top of the main peak of the monoclinic zirconia appears in XRD measurement, $I_t$ represents the integrated intensity of the peak near $2\theta = 30.3°$, where the peak top of the main peak of the tetragonal zirconia appears in XRD measurement, $I_c$ represents the integrated intensity of the peak near $2\theta = 30.0°$, where the peak top of the main peak of the cubic zirconia appears in XRD measurement, $I_d$ represents the integrated intensity of the peak near $2\theta = 29.6°$, where the peak top of the main peak of $Y_4Zr_3O_{12}$ appears in XRD measurement, and $I_y$ represents the integrated intensity of the peak near $2\theta = 29.2°$, where the peak top of the main peak of undissolved yttria appears in XRD measurement, and

given that the main peaks of $Y_4Zr_3O_{12}$, the tetragonal zirconia, and the cubic zirconia occur at similar positions, the integrated intensity of each main peak is calculated through peak separation.

[4] The zirconia pre-sintered body according to any one of [1] to [3], wherein the yttria content is 2.5 mol% or more and 10 mol% or less relative to the total moles of the zirconia and the yttria.

[5] A zirconia pre-sintered body comprising zirconia and yttria,

wherein, in comparison, the first translucency of a first sintered body fabricated by firing the zirconia pre-sintered body at 1,550°C for 7 minutes with a rate of temperature increase of 350°C/min is 85% or more of the second translucency of a second sintered body fabricated by firing the zirconia pre-sintered body at 1,550°C for 120 minutes with a rate of temperature increase of 10°C/min.

[6] A method for producing a zirconia pre-sintered body of any one of [1] to [5], comprising the steps of:

producing a zirconia composition that comprises zirconia particles and yttria particles;
pulverizing the zirconia composition;
molding the zirconia composition after pulverization to obtain a molded body; and
pre-sintering the molded body,
wherein the pulverization step involves (i) pulverization for a period of 10 minutes or more using a pulverization medium of less than 1 mm in diameter, or (ii) pulverization for a period of more than 40 hours using a pulverization medium of 1 mm or more in diameter.

[7] The method for producing a zirconia pre-sintered body according to [6], wherein the pre-sintering step involves a pre-sintering temperature of 600 to 1,300°C.

[8] A zirconia sintered body comprising zirconia, and a stabilizer capable of preventing a phase transformation of zirconia,

wherein at least one zirconia crystal grain contained in the zirconia sintered body has a region with a concentration gradient where the stabilizer concentration within the same crystal grain decreases from the central side of the crystal grain toward its outer crystal grain boundary.

[9] The zirconia sintered body according to [8], wherein the concentration gradient is 0.1 to 3 mol% relative to the total moles of the zirconia and the stabilizer.

[10] The zirconia sintered body according to [8] or [9], wherein the stabilizer capable of preventing a phase transformation of zirconia is yttria.

[11] A zirconia sintered body comprising zirconia, and a stabilizer capable of preventing a phase transformation of zirconia,

wherein the zirconia sintered body comprises:

a zirconia crystal grain (L) in which the stabilizer content is 4 to 6 mol% relative to the total moles of the zirconia and the stabilizer; and

a zirconia crystal grain (S) in which the stabilizer content is 1 to 3 mol% relative to the total moles of the zirconia and the stabilizer.

[12] The zirconia sintered body according to [11], wherein:

the zirconia crystal grain (L) has an average crystal grain diameter in a range of 0.5 to 2.0 $\mu$m, and
the zirconia crystal grain (S) has an average crystal grain diameter in a range of 0.1 to 0.3 $\mu$m.

[13] The zirconia sintered body according to [11] or [12], wherein the stabilizer capable of preventing a phase transformation of zirconia is yttria.

Advantageous Effects of Invention

According to the present invention, a zirconia pre-sintered body can be provided that can yield a zirconia sintered body with excellent translucency even with a hold time of 2 minutes at the highest sintering temperature. The present invention can also provide a method for producing such a zirconia pre-sintered body.

By using a zirconia pre-sintered body, a zirconia composition, and methods of their production of the present invention, a zirconia sintered body can be provided that can maintain high translucency comparable to that achieved by long sintering, even when sintered for a short period with a hold time of 7 minutes or less at 1,550°C. This helps improve productivity, for example by reducing treatment time at the dental clinic, making the invention advantageous for enabling one-visit treatments in dental clinics.

With a zirconia pre-sintered body, a zirconia composition, and methods of their production of the present invention, it is also possible to provide a zirconia sintered body that exhibits excellent mechanical strength and translucency with a lower highest sintering temperature (for example, 1,450°C or less) than that conventionally employed.

The present invention also enables the production of a zirconia sintered body that exhibits excellent translucency even when produced through a method that fires a zirconia pre-sintered body with a hold time of 2 minutes at the highest sintering temperature.

The present invention also enables the production of a zirconia sintered body that exhibits excellent mechanical strength even when produced through a method that fires a zirconia pre-sintered body with a hold time of 2 minutes at the highest sintering temperature.

BRIEF DESCRIPTION OF DRAWINGS

[FIG. 1] An electron diffraction image illustrating a method for calculating the distances and angles of diffraction spots.
[FIG. 2] A result of observation of a zirconia sintered body according to the present invention using a transmission electron microscope (TEM).

DESCRIPTION OF EMBODIMENTS

[Zirconia Pre-Sintered Body]

A zirconia pre-sintered body of the present invention comprises zirconia and yttria, along with $Y_4Zr_3O_{12}$ as a

crystal system. In other words, the present invention relates to a zirconia pre-sintered body that comprises zirconia and yttria components, along with $Y_4Zr_3O_{12}$ contained as a crystal system.

In this specification, "$Y_4Zr_3O_{12}$" refers to a metastable crystal system of zirconia present in the zirconia pre-sintered body. It is known that $Y_4Zr_3O_{12}$ exists up to around 1,375°C in the phase diagram.

As used herein, a "zirconia composition" refers to a composition comprising a zirconia powder and a yttria powder.

As used herein, a "molded body" refers to an object that has not reached a semi-sintered state (pre-sintered state) or a sintered state. That is, "molded body" distinguishes itself from pre-sintered body and sintered body in that it remains unfired after being molded.

As used herein, a "zirconia pre-sintered body" refers to an object in a semi-sintered state where zirconia particles have formed necks between particles but have not fully sintered.

As used herein, a "zirconia sintered body" refers to an object in a sintered state where zirconia particles have fully sintered. In a zirconia sintered body, sintering has caused zirconia particles to consolidate, increasing the relative density and promoting densification, resulting in a fully sintered state with a relative density of 95% or more.

As used herein, "zirconia" refers to zirconium(IV) oxide ($ZrO_2$), with $ZrO_2$ particles containing a trace amount (0.5 mass% or more and 3 mass% or less) of $HfO_2$ relative to the amount of $ZrO_2$. Because $HfO_2$ is difficult to separate, terms such as "zirconia", "zirconia particles", and "zirconia powder" are intended to include both $ZrO_2$ and $HfO_2$. Likewise, particles or powders with yttria present in zirconia as a solid solution are also encompassed by "zirconia particles" or "zirconia powders."

As used herein, the term "ordinary pressure" means standard atmospheric pressure (1 atm).

In this specification, the upper limits and lower limits of numeric ranges (for example, such as temperature ranges, the content of each component, the abundance of crystal systems, values calculated from components, and various physical properties) can be appropriately combined.

A certain preferred embodiment is, for example, a zirconia pre-sintered body in which the abundance $f_d$ (%) of $Y_4Zr_3O_{12}$ is 2.5% or more as calculated by the following formula (1-1).

$$f_d\ (\%) = I_d\ /\ (I_m + I_t + I_c + I_y + I_d) \times 100 \qquad (1\text{-}1),$$

wherein $f_d$ represents the abundance (%) of $Y_4Zr_3O_{12}$, $I_m$ represents the integrated intensity of the peak near $2\theta = 28.2°$, where the peak top of the main peak of the monoclinic zirconia appears in XRD measurement, $I_t$ represents the integrated intensity of the peak near $2\theta = 30.3°$, where the peak top of the main peak of the tetragonal zirconia appears in XRD measurement, $I_c$ represents the integrated intensity of the peak near $2\theta = 30.0°$, where the peak top of the main peak of the cubic zirconia appears in XRD measurement, $I_d$ represents the integrated intensity of the peak near $2\theta = 29.6°$, where the peak top of the main peak of $Y_4Zr_3O_{12}$ appears in XRD measurement, and $I_y$ represents the integrated intensity of the peak near $2\theta = 29.2°$, where the peak top of the main peak of undissolved yttria appears in XRD measurement.

Given that the main peaks of $Y_4Zr_3O_{12}$, the tetragonal zirconia, and the cubic zirconia occur at similar positions, the integrated intensity of each main peak is calculated through peak separation.

In the zirconia pre-sintered body according to the foregoing preferred embodiment, the abundance $f_d$ (%) of $Y_4Zr_3O_{12}$ is preferably 3.0% or more, more preferably 4.0% or more, even more preferably 5.0% or more, particularly preferably 6.0% or more. In view of providing a zirconia sintered body that exhibits excellent translucency even with a hold time of 2 minutes at the highest sintering temperature, or that can more easily maintain high translucency comparable to that achieved by long sintering even when sintered for a short period with a hold time of 7 minutes or less at 1,550°C, the abundance $f_d$ (%) of $Y_4Zr_3O_{12}$ is most preferably 6.5% or more. Considering that the phase transformation of $Y_4Zr_3O_{12}$ into other crystal systems becomes the rate-limiting step and impedes sintering of the zirconia pre-sintered body, the abundance $f_d$ (%) of $Y_4Zr_3O_{12}$ is preferably 30% or less, more preferably 25% or less, even more preferably 22% or less, particularly preferably 20% or less.

The abundance $f_d$ (%) of $Y_4Zr_3O_{12}$ can be calculated using the formula (1-1) above.

The abundance $f_d$ (%) of $Y_4Zr_3O_{12}$ can be measured using the method described in the EXAMPLES below.

By comprising $Y_4Zr_3O_{12}$ as a crystal system (particularly preferably when its abundance $f_d$ (%) falls within a predetermined range), a zirconia pre-sintered body of the present invention can yield a zirconia sintered body that exhibits excellent translucency even with a hold time of 2 minutes at the highest sintering temperature, or that can maintain high translucency comparable to that achieved by long sintering even when sintered for a short period with a hold time of 7 minutes or less at 1,550°C. Although the underlying reason is not fully understood, the following speculation has been made.

Incorporating $Y_4Zr_3O_{12}$ in the zirconia pre-sintered body reduces the uneven distribution of yttrium atoms in the pre-sintered body. This brings the yttrium atom distribution in the unsintered pre-sintered body closer to the final state of yttrium atoms that will be present in the sintered body, shortening the migration distance of yttrium atoms toward zirconia during firing, thereby reducing the heat energy required for firing. This probably explains why the resulting sintered body can exhibit translucency comparable to that achieved by conventional techniques, even when fired for a shorter period.

The zirconia pre-sintered body comprises yttria ($Y_2O_3$) as a stabilizer capable of preventing a phase transformation of zirconia. In this specification, the yttria contained as a component of the zirconia pre-sintered body does not necessarily need to exist solely as $Y_2O_3$, and may be present in a state containing yttrium elements (for example, such as $Y_4Zr_3O_{12}$). It is also not necessarily required that yttria be detectable as crystalline $Y_2O_3$.

The yttria content in the zirconia pre-sintered body is preferably 2.5 mol% or more, more preferably 3.0 mol% or more, even more preferably 3.5 mol% or more, particularly preferably 4.0 mol% or more relative to the total moles of zirconia (zirconium(IV) oxide, or $ZrO_2$) and yttria. A yttria content of 2.5 mol% or more is preferred in terms of increasing the cubic zirconia within the crystal systems of the sintered body and improving translucency.

The yttria content is preferably 10 mol% or less, more preferably 9.0 mol% or less, even more preferably 8.5 mol% or less, particularly preferably 8.0 mol% or less. A yttria content of 10 mol% or less is preferred in terms of preventing a decrease in mechanical strength. The stabilizer content in a zirconia pre-sintered body of the present invention can be measured using techniques, for example, such as inductively coupled plasma (ICP) emission spectral analysis, and X-ray fluorescence analysis.

In a zirconia pre-sintered body of the present invention, the stabilizer capable of preventing a phase transformation of zirconia may be solely yttria. Alternatively, a zirconia pre-sintered body of the present invention may additionally comprise another stabilizer, other than yttria, capable of preventing a phase transformation of zirconia.

Examples of such non-yttria stabilizers capable of preventing a phase transformation of zirconia (hereinafter, also referred to simply as "stabilizers") include oxides such as calcium oxide (CaO), magnesium oxide (MgO), cerium oxide ($CeO_2$), scandium oxide ($Sc_2O_3$), niobium oxide ($Nb_2O_5$), lanthanum oxide ($La_2O_3$), erbium oxide ($Er_2O_3$), praseodymium oxide ($Pr_2O_3$, $Pr_6O_{11}$), samarium oxide ($Sm_2O_3$), europium oxide ($Eu_2O_3$), thulium oxide ($Tm_2O_3$), gallium oxide ($Ga_2O_3$), indium oxide ($In_2O_3$), and ytterbium oxide ($Yb_2O_3$). The stabilizer may be used alone, or two or more thereof may be used in combination.

In a zirconia pre-sintered body of the present invention, in view of achieving superior translucency with $Y_4Zr_3O_{12}$ when the hold time at the highest sintering temperature is even shorter (for example, 2 minutes), it is preferable that the zirconia comprise monoclinic zirconia, and that the monoclinic fraction $f_m$ (%), calculated using formula (1-2), be less than 55%.

$$f_m\ (\%) = I_m\ /\ (I_m + I_t + I_c + I_y + I_d) \times 100 \qquad (1\text{-}2),$$

wherein $f_m$ represents the monoclinic fraction (%), $I_m$ represents the integrated intensity of the peak near $2\theta = 28.2°$, where the peak top of the main peak of the monoclinic zirconia appears in XRD measurement, $I_t$ represents the integrated intensity of the peak near $2\theta = 30.3°$, where the peak top of the main peak of the tetragonal zirconia appears in XRD measurement, $I_c$ represents the integrated intensity of the peak near $2\theta = 30.0°$, where the peak top of the main peak of the cubic zirconia appears in XRD measurement, $I_d$ represents the integrated intensity of the peak near $2\theta = 29.6°$, where the peak top of the main peak of $Y_4Zr_3O_{12}$ appears in XRD measurement, and $I_y$ represents the integrated intensity of the peak near $2\theta = 29.2°$, where the peak top of the main peak of undissolved yttria appears in XRD measurement.

Given that the main peaks of $Y_4Zr_3O_{12}$, the tetragonal zirconia, and the cubic zirconia occur at similar positions, the integrated intensity of each main peak is calculated through peak separation.

In view of achieving superior translucency in the zirconia sintered body through the combined effect of $Y_4Zr_3O_{12}$ even with a hold time of 2 minutes at the highest sintering temperature, the monoclinic fraction $f_m$ (%) is more preferably 48% or less, even more preferably 46% or less, particularly preferably 45% or less.

The monoclinic fraction $f_m$ (%) is preferably 5% or more, more preferably 10% or more, even more preferably 15% or more, particularly preferably 20% or more.

In view of achieving superior translucency with $Y_4Zr_3O_{12}$ when the hold time at the highest sintering temperature is even shorter (for example, 2 minutes), another preferred embodiment is, for example, a zirconia pre-sintered body that comprises $Y_4Zr_3O_{12}$ as a crystal system, and in which the monoclinic fraction $f_m$ (%) is 0% or more and less than 5%.

The zirconia pre-sintered body in such an embodiment is not particularly limited, and may be tetragonal zirconia or cubic zirconia, provided that the monoclinic fraction $f_m$ (%) is 0% or more and less than 5%.

A zirconia pre-sintered body of the present invention may comprise additives other than zirconia and stabilizers, provided that the present invention can exhibit its effects. Examples of such additives include colorants (including pigments, composite pigments, and fluorescent agents), alumina ($Al_2O_3$), titanium oxide ($TiO_2$), and silica ($SiO_2$).

Examples of the pigments include oxides of at least one element selected from the group consisting of Ti, V, Cr, Mn, Fe, Co, Ni, Zn, Y, Sn, Sb, Bi, Ce, Pr, Sm, Eu, Gd, Tb, and Er (specifically, such as NiO and $Cr_2O_3$), excluding $Y_2O_3$ and $CeO_2$, preferably oxides of at least one element selected from the group consisting of Ti, V, Cr, Mn, Fe, Co, Ni, Zn, Y, Sn, Sb, Bi, Ce, Pr, Sm, Eu, Gd, and Tb, more preferably oxides of at least one element selected from the group consisting of Ti, V, Cr, Mn, Fe, Co, Ni, Zn, Y, Sn, Sb, Bi, Ce, Sm, Eu, Gd, and Tb.

A zirconia pre-sintered body of the present invention may be one that does not comprise erbium oxide ($Er_2O_3$).

Examples of the composite pigments include $(Zr,V)O_2$, $Fe(Fe,Cr)_2O_4$, $(Ni,Co,Fe)(Fe,Cr)_2O_4 \cdot ZrSiO_4$, and $(Co,Zn)Al_2O_4$. Examples of the fluorescent agents include $Y_2SiO_5$:Ce, $Y_2SiO_5$:Tb, $(Y,Gd,Eu)BO_3$, $Y_2O_3$:Eu, YAG:Ce, $ZnGa_2O_4$:Zn, and $BaMgAl_{10}O_{17}$:Eu.

A zirconia pre-sintered body of the present invention may comprise a fluorescent agent. The zirconia sintered body can exhibit fluorescence by incorporating a fluorescent agent in the zirconia pre-sintered body. The type of fluorescent agent is not particularly limited, and it is possible to use a single fluorescent agent or two or more fluorescent agents capable of emitting fluorescence at any wavelength of light.

Examples of the fluorescent agent include those containing metal elements. Examples of the metal elements include Ga, Bi, Ce, Nd, Sm, Eu, Gd, Tb, Dy, and Tm. The fluorescent agent may contain one of these metal elements by itself, or may contain two or more of these metal elements. Preferred among these metal elements are Ga, Bi, Eu, Gd, and Tm, more preferably Bi and Eu.

Examples of such fluorescent agents include oxides, hydroxides, acetates, and nitrates of the metal elements above. The fluorescent agent may be, for example, $Y_2SiO_5$:Ce, $Y_2SiO_5$:Tb, $(Y,Gd,Eu)BO_3$, $Y_2O_3$:Eu, YAG:Ce, $ZnGa_2O_4$:Zn, or $BaMgAl_{10}O_{17}$:Eu.

The content of the fluorescent agent in the zirconia pre-sintered body is not particularly limited, and may be appropriately adjusted according to factors such as the type of fluorescent agent, and the intended use of the zirconia sintered body. However, in view of considerations such as suitability as dental prostheses, the content of fluorescent agent is preferably 0.001 mass% or more, more preferably 0.005 mass% or more, even more preferably 0.01 mass% or more in terms of the oxide equivalent of the metal elements contained in the fluorescent agent, relative to 100 mass% of the zirconia contained in the zirconia pre-sintered body.

The content of fluorescent agent is not particularly limited, and may be 1 mass% or less, 0.5 mass% or less, or 0.1 mass% or less in terms of the oxide equivalent of the metal elements contained in the fluorescent agent, provided that the fluorescent agent can exhibit suitable fluorescence. When the content is at or above these lower limits, the fluorescence can match or exceed that of natural human teeth. When the content is at or below the foregoing upper limits, it is possible to reduce a decrease in translucency and mechanical strength.

A zirconia pre-sintered body of the present invention can yield a zirconia sintered body that exhibits excellent translucency even with a hold time of 7 minutes or less (for example, 2 minutes) at 1,550°C.

The sintered body fabricated by firing the zirconia pre-sintered body at 1,550°C for 7 minutes with a rate of temperature increase of 350°C/min is denoted as a first sintered body. Similarly, the sintered body fabricated by firing a zirconia pre-sintered body-identical in size to the zirconia pre-sintered body used to produce the first sintered body-at 1,550°C for 120 minutes with a rate of temperature increase of 10°C/min is denoted as a second sintered body.

A certain preferred embodiment of the present invention is, for example, a zirconia pre-sintered body comprising zirconia and yttria, wherein, in comparison, the first translucency of a first sintered body fabricated by firing the zirconia pre-sintered body at 1,550°C for 7 minutes with a rate of temperature increase of 350°C/min is 85% or more of the second translucency of a second sintered body fabricated by firing the zirconia pre-sintered body at 1,550°C for 120 minutes with a rate of temperature increase of 10°C/min.

When the first sintered body fabricated with a firing time of 7 minutes at 1,550°C is compared with the second sintered body with respect to translucency, the translucency of the first sintered body is preferably 85% or more, more preferably 90% or more, even more preferably 95% or more of the translucency of the second sintered body, particularly preferably essentially the same as the translucency of the second sintered body.

As described above, a zirconia pre-sintered body of the present invention has an advantage with respect to the short firing period of 7 minutes or less.

The method for measuring translucency will be described in the EXAMPLES below.

Another certain preferred embodiment of the present invention is, for example, a zirconia pre-sintered body that comprises zirconia, yttria, and $Y_4Zr_3O_{12}$, and in which the translucency of the first sintered body falls within the foregoing preferred ranges (for example, 85% or more) relative to the translucency of the second sintered body.

In order to ensure mechanical strength that enables mechanical processing in a pre-sintered state, the flexural strength of a zirconia pre-sintered body of the present invention is preferably 15 MPa or more. For ease of mechanical processing in a pre-sintered state, the flexural strength of the zirconia pre-sintered body is preferably 70 MPa or less, more preferably 60 MPa or less.

The flexural strength can be measured in compliance with ISO 6872:2015 (Dentistry-Ceramic materials). For measurement, specimens measuring 5 mm × 10 mm × 50 mm are used under the same conditions except for size. For surface finishing, the specimen surfaces, including the chamfered surface (45° chamfers at the corners of specimen), are finished longitudinally with #600 sandpaper. The specimen is disposed in such an orientation that its widest face is perpendicular to the vertical direction (loading direction). In the three-point flexural test, measurement is carried out at a crosshead speed of 0.5 mm/min with the distance between supports (span) set at 30 mm.

A zirconia pre-sintered body of the present invention has a density of preferably 2.7 g/cm$^3$ or more, more preferably 3.0 g/cm$^3$ or more, even more preferably 3.2 g/cm$^3$ or more.

The zirconia pre-sintered body has a density of preferably 4.0 g/cm$^3$ or less, more preferably 3.8 g/cm$^3$ or less, even more preferably 3.6 g/cm$^3$ or less. A density in these ranges allows for easy molding.

The density of the zirconia pre-sintered body can be calculated as the mass-to-volume ratio (mass of zirconia pre-sintered body) / (volume of zirconia pre-sintered body). For example, the density of the zirconia pre-sintered body can be determined by cutting out test specimens with dimensions of 10 mm × 10 mm × 10 mm from arbitrary locations of the zirconia pre-sintered body (n = 3), measuring the mass and volume of each test specimen, and averaging the measured values. The calculated arithmetic average can then be applied to the above formula to determine the density.

[Production Method of Zirconia Pre-Sintered Body]

The following describes a method for producing the zirconia pre-sintered body.

A zirconia pre-sintered body of the present invention can be produced by pulverizing a zirconia composition, and firing (pre-sintering) a molded body of the pulverized zirconia composition to an extent that the zirconia particles do not sinter, with the pulverization process providing excess energy under predetermined conditions compared to conventional techniques.

An example of a method for producing a zirconia pre-sintered body of the present invention comprises the steps of:

producing a zirconia composition that comprises zirconia particles and yttria particles;
pulverizing the zirconia composition;
molding the zirconia composition after pulverization to obtain a molded body; and
pre-sintering the molded body,
wherein the pulverization step includes the predetermined pulverization process described below.

The zirconia composition can be produced by mixing the raw material zirconia powder and yttria powder. The mixing method is not particularly limited, and any known method and apparatus may be used.

The zirconia and yttria particles constituting their respective powders may be prepared using a method, for example, such as the breakdown process, in which coarse particles are pulverized or crushed into a fine powder, or the building-up process, which synthesizes particles through nucleation and growth from atoms or ions.

The zirconia constituting the zirconia powder is not particularly limited, and may be any of tetragonal zirconia, monoclinic zirconia, or cubic zirconia.

These may be present alone, or two or more thereof may be present in combination in the zirconia powder.

The zirconia powder may employ commercially available zirconia particles, or a commercially available powder may be used after pulverization with a known pulverizing mixer (such as a ball mill).

The yttria powder may employ commercially available yttria particles, or a commercially available powder may be used after pulverization with a known pulverizing mixer (such as a ball mill).

Examples of commercially available zirconia powders include zirconia powder products such as those manufactured by Tosoh Corporation under various trade names, including Zpex® (Y$_2$O$_3$ content: 3 mol%), Zpex® 4 (Y$_2$O$_3$ content: 4 mol%), Zpex® Smile® (Y$_2$O$_3$ content: 5.5 mol%), TZ-3Y (Y$_2$O$_3$ content: 3 mol%), TZ-3YS (Y$_2$O$_3$ content: 3 mol%), TZ-4YS (Y$_2$O$_3$ content: 4 mol%), TZ-6Y (Y$_2$O$_3$ content: 6 mol%), TZ-6YS (Y$_2$O$_3$ content: 6 mol%), TZ-8YS (Y$_2$O$_3$ content: 8 mol%), TZ-10YS (Y$_2$O$_3$ content: 10 mol%), TZ-3Y-E (Y$_2$O$_3$ content: 3 mol%), TZ-3YS-E (Y$_2$O$_3$ content: 3 mol%), TZ-3YB-E (Y$_2$O$_3$ content: 3 mol%), TZ-3YSB-E (Y$_2$O$_3$ content: 3 mol%), TZ-3YB (Y$_2$O$_3$ content: 3 mol%), TZ-3YSB (Y$_2$O$_3$ content: 3 mol%), TZ-3Y20AB (Y$_2$O$_3$ content: 3 mol%), TZ-8YSB (Y$_2$O$_3$ content: 8 mol%), and TZ-0 (Y$_2$O$_3$ content: 0 mol%; monoclinic zirconia).

The zirconia composition is pulverized further. By providing excess energy in the pulverization process under predetermined conditions compared to conventional techniques, the resulting zirconia composition can generate Y$_4$Zr$_3$O$_{12}$ when subjected to pre-sintering.

Zirconia itself is a material with notably high hardness. Accordingly, increasing the energy applied in a pulverization process does not readily make the zirconia particles excessively small. From this perspective, excessive pulverization has not been considered to have technical significance.

However, by subjecting a zirconia composition comprising zirconia and yttria to a pulverization process, and applying excess energy in the pulverization process compared to conventional techniques (i.e., when pulverization is carried out under applied energy in excess of that conventionally employed while both zirconia and yttria are present), the zirconia composition can generate Y$_4$Zr$_3$O$_{12}$ when prepared into a pre-sintered body. This is considered to underlie the ability of the present invention to achieve superior translucency even with a hold time of 2 minutes at the highest sintering temperature.

Preferably, the pulverization process employs the following method.

(i) pulverization using a pulverization medium of less than 1 mm in diameter, or
(ii) pulverization for a period of more than 40 hours using a pulverization medium of 1 mm or more in diameter.

Pulverization media with fine diameters help generate $Y_4Zr_3O_{12}$ when producing a pre-sintered body. From this viewpoint, when using a pulverization medium of less than 1 mm in diameter, the size (diameter) of the pulverization medium is preferably 0.1 to 0.5 mm.

The pulverization medium of less than 1 mm in diameter may be a commercially available product.

The pulverization medium of less than 1 mm in diameter may be used with a pulverization device, for example, such as a bead mill.

When using a pulverization medium of less than 1 mm in diameter, the duration of the pulverization process is not particularly limited. However, in view of more readily generating $Y_4Zr_3O_{12}$ when producing a pre-sintered body, the pulverization process is preferably 10 minutes or more, more preferably 15 minutes or more, even more preferably 20 minutes or more. The pulverization process is preferably 20 hours or less, more preferably 10 hours or less, even more preferably 5 hours or less, particularly preferably 2 hours or less.

When the pulverization device is a device that operates by circulating slurry (for example, a circulation bead mill), the duration of the pulverization process refers to the period for which the slurry remains in the vessel (pulverization chamber).

In view of providing a long pulverization time and more readily increasing the energy level in the pulverization process in combination with the extended pulverization time, the size (diameter) of pulverization medium is preferably 1.5 mm or more, more preferably 2.0 mm or more when using a pulverization medium of 1 mm or more in diameter.

The pulverization medium of 1 mm or more in diameter may be a commercially available product.

The pulverization medium of 1 mm or more in diameter may be used with a pulverization device, for example, such as a ball mill.

When using a pulverization medium of 1 mm or more in diameter, the pulverization time (the duration of the pulverization process) is not particularly limited. However, the pulverization time is preferably more than 40 hours, more preferably 55 hours or more. In view of allowing easier pulverization of yttria particles into fine particle sizes, and more readily increasing the abundance $f_d$ of $Y_4Zr_3O_{12}$ when producing a pre-sintered body, the pulverization time is even more preferably 80 hours or more, particularly preferably 100 hours or more. The duration of the pulverization process is preferably 1,000 hours or less, more preferably 800 hours or less, even more preferably 500 hours or less, particularly preferably 300 hours or less.

In the present invention, by applying increased pulverization energy adjusted through the combination of pulverization time and pulverization medium size, the resulting zirconia composition can generate $Y_4Zr_3O_{12}$ during pre-sintering.

It is believed that the zirconia composition that has received excess pulverization energy enables a zirconia pre-sintered body of the present invention to comprise $Y_4Zr_3O_{12}$ as a crystal system, and allows easier adjustment to predetermined fractions that are more effective.

The zirconia composition may have various forms, including granules and slurry.

When the zirconia composition is a slurry, it can be produced by mixing the mixed powder from the pulverization process with a solvent (preferably water).

In view of providing a sintered body having excellent translucency even with a hold time of 2 minutes at the highest sintering temperature, the average particle diameter of the zirconia composition after pulverization is preferably 0.2 μm or less.

The average particle diameter of zirconia particles and yttria particles in the zirconia composition after pulverization can be measured using a dynamic light scattering particle size distribution measurement method. For example, measurement may be conducted using a dynamic light scattering particle size distribution analyzer (manufactured by Horiba Ltd. under the trade name SZ-100V2), where a slurry diluted with water to about 0.1 mass% is subjected to ultrasonic waves for 30 minutes, and subsequently measured by volume under continued ultrasonic radiation.

The zirconia composition may additionally comprise additives such as binders, dispersants, emulsifiers, antifoaming agents, pH adjusters, lubricants, and translucency adjusters. The additives may be used alone, or two or more thereof may be used in combination.

Examples of the binders include polyvinyl alcohol, methyl cellulose, carboxymethyl cellulose, acrylic binders, wax binders, polyvinyl butyral, polymethyl methacrylate, and ethyl cellulose.

For improved translucency, the binder content in a zirconia composition of the present invention is preferably 10 mass% or less, more preferably 7 mass% or less, even more preferably 5 mass% or less relative to 100 mass% of zirconia.

Examples of the plasticizers include polyethylene glycol, glycerin, propylene glycol, and dibutyl phthalate.

Examples of the dispersants include ammonium polycarboxylate (such as triammonium citrate), ammonium polyacrylate, acryl copolymer resins, acrylic acid ester copolymers, polyacrylic acid, bentonite, carboxymethyl cellulose, anionic surfactants (for example, polyoxyethylene alkyl ether phosphoric acid esters such as polyoxyethylene lauryl ether phosphoric acid ester), non-ionic surfactants, oleic glyceride, amine salt surfactants, oligosaccharide alcohols, and stearic acid.

Examples of the emulsifiers include alkyl ethers, phenyl ethers, and sorbitan derivatives.

Examples of the antifoaming agents include alcohols, polyethers, polyethylene glycols, silicone, and waxes.

Examples of the pH adjusters include ammonia, ammonium salts (including ammonium hydroxides such as tetramethylammonium hydroxide).

Examples of the lubricants include polyoxyethylene alkyl ethers, and waxes.

Examples of the translucency adjusters include aluminum oxide ($Al_2O_3$), titanium oxide ($TiO_2$), silicon dioxide ($SiO_2$), zircon, lithium silicate, and lithium disilicate.

The particles constituting the zirconia composition have a BET specific surface area of preferably 7.0 $m^2$/g or more, more preferably 7.5 $m^2$/g or more, even more preferably 8.0 $m^2$/g or more as measured in compliance with JIS Z 8830:2013. When the BET specific surface area is 7.0 $m^2$/g or more, it is possible to inhibit opacification of the sintered body upon sintering. The BET specific surface area is preferably 50 $m^2$/g or less, more preferably 45 $m^2$/g or less, even more preferably 40 $m^2$/g or less. A BET specific surface area of 50 $m^2$/g or less reduces susceptibility to the influence of temperature variations inside the furnace.

The BET specific surface area can be measured using a commercially available product, such as a full automatic specific surface area analyzer (manufactured by Mountech Co., Ltd. under the trade name Macsorb® HM model-1200; BET flow method (single point / multi point)). For example, measurement may be conducted using this full automatic specific surface area analyzer following the BET flow method (single point).

The translucency of the sintered body also becomes less likely to decrease with a shorter firing period in sintering. Here, a "BET specific surface area" is a specific surface area measured without distinguishing primary particles and secondary particles.

The next step is the molding process, where the pulverized zirconia composition is molded to prepare a molded body. The molded body can be obtained by molding the zirconia composition under applied external force using a known method.

The molding method is not particularly limited, and may employ the following methods, for example.

(a) slip casting of a slurry containing the pulverized zirconia composition
(b) gel casting of a slurry containing the pulverized zirconia composition
(c) press molding of the pulverized zirconia composition
(d) molding of a zirconia composition containing zirconia particles, yttria particles, and resin
(e) polymerization of a zirconia composition containing zirconia particles, yttria particles, and a polymerizable monomer or oligomer
(f) additive fabrication of granules containing zirconia particles and yttria particles

(a) Slip Casting

When the zirconia molded body is produced using a method that includes the step of slip casting a slurry containing the pulverized zirconia composition, the slip casting is not particularly limited to specific methods, and may be carried out using, for example, a method that dries the slurry after pouring it in a mold.

For considerations such as ease of pouring the slurry into a mold, and increasing the reusability of the mold in addition to preventing excessive drying time, the content of the dispersion medium in the slurry used is preferably 80 mass% or less, more preferably 50 mass% or less, even more preferably 20 mass% or less.

The slurry may be poured into a mold under ordinary pressure. However, in view of production efficiency, it is preferable to pour the slurry under applied pressure conditions.

(b) Gel Casting

When the zirconia molded body is produced using a method that includes the step of gel casting a slurry containing the pulverized zirconia composition, the gel casting is not particularly limited to specific methods, and may be carried out using, for example, a method that dries a moist body shaped in a mold by gelling the slurry.

For considerations such as preventing excessive drying time and reducing cracking during drying, the content of the dispersion medium contained in the slurry used is preferably 80 mass% or less, more preferably 50 mass% or less, even more preferably 20 mass% or less.

The gelation may be achieved by, for example, adding a gelatinizer, or by adding and polymerizing a polymerizable monomer.

The gelatinizer is not particularly limited, and may be, for example, a water-soluble gelatinizer. Specifically, preferred are agarose and gelatin, for example. The gelatinizer may be used alone, or two or more thereof may be used in combination. The gelatinizer may be used in any amount, as long as it does not cause problems such as cracking during sintering. The gelatinizer may be used in an amount of 10 mass% or less, 5 mass% or less, or 1 mass% or less, based on the mass the slurry after the addition of gelatinizer.

The polymerizable monomer is not particularly limited, and may be, for example, 2-hydroxyethyl (meth)acrylate, 3-hydroxypropyl (meth)acrylate, 4-hydroxybutyl (meth)acrylate, 6-hydroxyhexyl (meth)acrylate, 10-hydroxydecyl (meth)acrylate, propylene glycol mono(meth)acrylate, glycerol mono(meth)acrylate, erythritol mono(meth)acrylate, N-methylol(meth)acrylamide, N-hydroxyethyl(meth)acrylamide, or N,N-bis(2-hydroxyethyl)(meth)acrylamide. The polymerizable monomer may be used alone, or two or more thereof may be used in combination.

The polymerizable monomer may be used in any amount, as long as it does not cause problems such as cracking during sintering. The polymerizable monomer may be used in an amount of 10 mass% or less, 5 mass% or less, or 1 mass% or less, based on the mass the slurry after the addition of polymerizable monomer.

When gelation is performed through polymerization of a polymerizable monomer, it is preferable to use a polymerization initiator for polymerization. The polymerization initiator is not particularly limited. Particularly preferred are photopolymerization initiators. The photopolymerization initiator can be appropriately selected from photopolymerization initiators used in industry, preferably those used in dentistry.

Specific examples of photopolymerization initiators include (bis)acylphosphine oxides (including salts), thioxanthones (including salts such as quaternary ammonium salts), ketals, $\alpha$-diketones, coumarins, anthraquinones, benzoin alkyl ether compounds, and $\alpha$-aminoketone compounds. The photopolymerization initiators may be used alone, or two or more thereof may be used in combination. Preferred among these photopolymerization initiators is at least one selected from the group consisting of (bis)acylphosphine oxides and $\alpha$-diketones. With these photopolymerization initiators, it is possible to achieve polymerization (gelation) both in the ultraviolet region (including the near ultraviolet region) and the visible region. Specifically, polymerization (gelation) can sufficiently take place irrespective of whether the light source used is a laser such as an Ar laser or a He-Cd laser, or a lamp such as a halogen lamp, a xenon lamp, a metal halide lamp, a light emitting diode (LED), a mercury lamp, or a fluorescent lamp.

The method used to dry the shaped moist body is not particularly limited, and may be, for example, natural drying, hot-air drying, vacuum drying, drying by dielectric heating, drying by induction heating, or drying under constant temperature and humidity. These methods may be used alone, or two or more thereof may be used in combination. In view of considerations such as the capability to reduce cracking during drying, preferred are natural drying, drying by dielectric heating, drying by induction heating, and drying under constant temperature and humidity.

The molds used for the slip casting and gel casting are not particularly limited, and may be, for example, porous molds made of a material such as plaster, resin, or ceramic, or non-porous molds made of a material such as metal or resin.

(c) Press Molding

In the press molding of the pulverized zirconia composition, the press molding is not particularly limited to specific methods, and may be carried out using a known press molding machine.

Specific examples of the press molding method include uniaxial pressing.

The press molding may be conducted in multiple stages. For example, the zirconia composition after press molding may be subjected to CIP (Cold Isostatic Pressing).

The shape of the molded body is not particularly limited, and may be disk-shaped, cuboidal, or the form of a dental product (for example, a crown shape).

The molded body may be, for example, a columnar zirconia molded body formed by filling a mold with the zirconia composition (for example, granules) and compacting it by uniaxial pressing.

The density of the molded body increases with higher surface pressure in press molding. However, the zirconia molded body yields a hard zirconia pre-sintered body when its density is too high. Accordingly, the surface pressure of press molding for the preparation of the zirconia molded body is preferably 30 to 200 MPa. When the surface pressure is 30 MPa or more, superior shape retention can be achieved in the zirconia molded body, whereas a surface pressure of 200 MPa or less prevents the density of the zirconia molded body from becoming too high and helps avoid excessive hardening more effectively.

The molded body includes those densified through high-temperature pressing such as CIP (Cold Isostatic Pressing).

From the same perspectives described above, the CIP pressure is preferably 30 to 200 MPa.

(d) Molding of Zirconia Composition Containing Resin

When the zirconia molded body is produced using a method that comprises the step of molding a zirconia composition containing zirconia particles, yttria particles, and resin, the method used to mold the zirconia composition is not particularly limited to specific methods, and may be, for example, injection molding, cast molding, or extrusion molding.

It is also possible to use additive fabrication techniques (such as 3D printing) to form the composition, including, for example, fused deposition modeling (FDM), the inkjet method, and the powder/binder lamination method. Preferred among these molding methods are injection molding and cast molding, more preferably injection molding.

The resin is not particularly limited, and is preferably the binder described above.

(e) Polymerization of Zirconia Composition Containing Zirconia Particles, Yttria Particles, and Polymerizable Monomer or Oligomer

By polymerizing a zirconia composition containing zirconia particles, yttria particles, and a polymerizable monomer or oligomer, the polymerizable monomer or oligomer in the zirconia composition can polymerize, allowing the composition to cure.

When the zirconia molded body is produced using a method that comprises such a polymerization step, the method is not particularly limited to specific methods, and may be, for example, a method by which a zirconia composition is polymerized in a mold, or stereolithography (SLA) using a zirconia composition. The preferred method is (b) stereolithography (SLA).

Stereolithography enables the zirconia molded body to have a shape corresponding to the shape desired for the final zirconia sintered body, at the time of its production. This makes stereolithography a potentially preferred method in certain situations, particularly when the zirconia sintered body is used as a dental material such as a dental prosthesis.

The polymerizable monomer is not particularly limited, and may be a monofunctional polymerizable monomer such as a monofunctional (meth)acrylate or a monofunctional (meth)acrylamide, or a polyfunctional polymerizable monomer such as a bifunctional aromatic compound, a bifunctional aliphatic compound, or a tri- or higher-functional compound. The polymerizable monomer may be used alone, or two or more thereof may be used in combination. Preferred for use are polyfunctional polymerizable monomers, particularly in situations where stereolithography is employed.

The oligomer is not particularly limited, as long as it is a polymerizable compound formed by the bonding of two or more of the aforementioned polymerizable monomers.

Examples of the monofunctional (meth)acrylate include:

(meth)acrylates having a hydroxyl group(s), such as 2-hydroxyethyl (meth)acrylate, 2-hydroxypropyl (meth)acrylate, 3-hydroxypropyl (meth)acrylate, 4-hydroxybutyl (meth)acrylate, 6-hydroxyhexyl (meth)acrylate, 10-hydroxydecyl (meth)acrylate, propylene glycol mono(meth)acrylate, glycerol mono(meth)acrylate, and erythritol mono(meth)acrylate;

alkyl (meth)acrylates such as methyl (meth)acrylate, ethyl (meth)acrylate, propyl (meth)acrylate, isopropyl (meth)acrylate, n-butyl (meth)acrylate, sec-butyl (meth)acrylate, t-butyl (meth)acrylate, isobutyl (meth)acrylate, n-hexyl (meth)acrylate, lauryl (meth)acrylate, cetyl (meth)acrylate, and stearyl (meth)acrylate;

alicyclic (meth)acrylates such as cyclohexyl (meth)acrylate, and isobornyl (meth)acrylate;

aromatic group-containing (meth)acrylates such as benzyl (meth)acrylate, and phenyl (meth)acrylate; and

(meth)acrylates having functional groups, such as 2,3-dibromopropyl (meth)acrylate, 3-(meth)acryloyloxypropyltrimethoxysilane, and 11-(meth)acryloyloxyundecyltrimethoxysilane.

Examples of the monofunctional (meth)acrylamide include (meth)acrylamide, N-(meth)acryloylmorpholine, N,N-dimethyl(meth)acrylamide, N,N-diethyl(meth)acrylamide, N,N-di-n-propyl(meth)acrylamide, N,N-di-n-butyl(meth)acrylamide, N,N-di-n-hexyl(meth)acrylamide, N,N-di-n-octyl(meth)acrylamide, N,N-di-2-ethylhexyl(meth)acrylamide, N-hydroxyethyl(meth)acrylamide, and N,N-bis(2-hydroxyethyl)(meth)acrylamide.

In view of excellent polymerizability, preferred among these monofunctional polymerizable monomers are (meth)acrylamides, more preferably N-(meth)acryloylmorpholine, N,N-dimethyl(meth)acrylamide, and N,N-diethyl(meth)acrylamide.

Examples of the bifunctional aromatic compound include (meth)acrylates such as 2,2-bis((meth)acryloyloxyphenyl)propane, 2,2-bis[4-(2-hydroxy-3-acryloyloxy-2-hydroxypropoxy)phenyl]propane, 2,2-bis[4-(2-hydroxy-3-methacryloyloxypropoxy)phenyl]propane (commonly known as Bis-GMA), 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, and 1,4-bis(2-(meth)acryloyloxyethyl)pyromellitate. In view of excellent polymerizability and providing a zirconia molded body having superior mechanical strength, preferred are Bis-GMA, and 2,2-bis(4-(meth)acryloyloxypolyethoxyphenyl)propane. Preferred as 2,2-bis(4-(meth)acryloyloxypolyethoxyphenyl)propane is 2,2-bis(4-methacryloyloxypolyethoxyphenyl)propane (with an average of 2.6 moles of ethoxy group added; commonly known as D-2.6E).

Examples of the bifunctional aliphatic compound include (meth)acrylates such as glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,4-butanediol di(meth)acrylate, 1,6-hexanediol di(meth)acrylate, 2-ethyl-1,6-hexanediol di(meth)acrylate, 1,9-nonanediol di(meth)acrylate, 1,10-decanediol di(meth)acrylate, 1,2-bis(3-methacryloyloxy-2-hydroxypropoxy)ethane, and 2,2,4-trimethylhexamethylenebis(2-carbamoyloxyethyl)dimethacrylate (commonly known as UDMA). In view of excellent polymerizability and providing a zirconia molded body having superior mechanical strength, preferred are triethylene glycol dimethacrylate (commonly known as TEGDMA), and UDMA.

Examples of the tri- or higher-functional compound include (meth)acrylates such as trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol]tetra(meth)acrylate, and 1,7-diacryloyloxy-2,2,6,6-tetra(meth)acryloyloxymethyl-4-oxaheptane. In view of excellent polymerizability and providing a zirconia molded body having superior mechanical strength, preferred are N,N-(2,2,4-trimethylhexamethylene)bis[2-(aminocarboxy)propane-1,3-diol]tetramethacrylate, and 1,7-diacryloyloxy-2,2,6,6-tetraacryloyloxymethyl-4-oxaheptane.

In both of the foregoing methods, it is preferable that the composition be polymerized using a polymerization initiator, and that the composition additionally comprise a polymerization initiator. The polymerization initiator is not particularly limited. Particularly preferred are photopolymerization initiators. The photopolymerization initiator may be appropriately selected from photopolymerization initiators used in industry, preferably those used in dentistry. Specific examples of the photopolymerization initiator are no different from those mentioned above in the description of gel casting.

When the zirconia molded body is produced by stereolithography using the zirconia composition, the stereolithography method is not particularly limited to specific methods, and a known method may be appropriately selected for stereolithography. For example, it is possible to use a method that produces the desired zirconia molded body by successively forming layers in desired shapes through photopolymerization of a liquid composition with, for example, ultraviolet light or a laser, using a stereolithography device.

The zirconia molded body, as the cured product, may be subjected to a CIP process after a humidification process, in order to further improve the density of the zirconia molded body.

When press molding is performed, the zirconia particle-containing powder may be subjected to press molding after a humidification process, before pressing the powder. Any known methods can be used for the humidification process, including a method that sprays water with a sprayer or the like, and a method that uses a constant humidity chamber or a constant temperature and humidity chamber. The amount of moisture increase after the humidification process is preferably more than 2 mass%, more preferably more than 3 mass%, even more preferably more than 4 mass%, particularly preferably more than 5 mass%, and is preferably 15 mass% or less, more preferably 13 mass% or less, even more preferably 11 mass% or less relative to the mass of the unwetted powder (powder before humidification process) and molded body, though the amount of moisture increase depends on factors such as the average particle size of the zirconia particles and stabilizer particles contained. The amount of moisture increase by humidification process can be determined as a percentage by subtracting the mass of the unwetted powder and molded body from the mass of the wetted powder (powder after humidification process) and molded body, and dividing the difference by the mass of the unwetted powder and molded body.

The CIP process uses the same pressure as specified previously for press molding.

(f) Additive Fabrication of Granules Containing Zirconia Particles and Yttria Particles

There is no specific restriction on the method for producing granules containing zirconia particles and yttria particles. For example, granules can be obtained by preparing a slurry and drying it with a spray dryer, and these granules may be used for powder additive fabrication.

The technique employed for powder additive fabrication is not particularly limited. Examples include the powder bed method, SLS method (selective laser sintering), SLM method (selective laser melting), electron beam method, arc discharge method, and binder jet method. For techniques where organic materials should be excluded in additive fabrication, it is preferable to also avoid using organic materials during the production of granules.

The next step is pre-sintering of the molded body to obtain a zirconia pre-sintered body of the present invention.

In view of obtaining $Y_4Zr_3O_{12}$ and ensuring a semi-sintered state using the specific zirconia composition above, the pre-sintering temperature (highest pre-sintering temperature) in the pre-sintering step is preferably 600°C or more, more preferably 700°C or more, even more preferably 800°C or more, particularly preferably 850°C or more.

In view of obtaining $Y_4Zr_3O_{12}$ and ensuring processability, the pre-sintering temperature is preferably 1,300°C or less, more preferably 1,250°C or less, even more preferably 1,200°C or less, particularly preferably 1,150°C or less.

That is, the preferred pre-sintering temperature is 600 to 1,300°C in the method of production of a zirconia pre-sintered body of the present invention.

The hold time (retention time) at the highest pre-sintering temperature is not particularly limited, as long as it can achieve a semi-sintered state and yield a zirconia pre-sintered body comprising $Y_4Zr_3O_{12}$. Preferably, the highest pre-sintering temperature is held for 30 minutes to 6 hours.

The rate of temperature increase to the highest pre-sintering temperature, and the rate of temperature decrease from the highest pre-sintering temperature are preferably 300°C/min or less.

In certain preferred embodiments, the rate of temperature increase to the highest pre-sintering temperature in pre-sintering of a zirconia molded body of the present invention is not particularly limited. However, the rate of temperature increase to the highest pre-sintering temperature is preferably 0.1°C/min or more, more preferably 0.2°C/min or more, even more preferably 0.5°C/min or more, and is preferably 50°C/min or less, more preferably 30°C/min or less, even more preferably 20°C/min or less.

The productivity improves when the rate of temperature increase is at or above these lower limits. When the rate of temperature increase is at or below the foregoing upper limits, it is possible to reduce the volume difference between the inner and outer portions of the zirconia molded body and zirconia pre-sintered body. Additionally, when the zirconia molded body contains organic materials, the rapid decomposition of these organic materials can be reduced to inhibit cracks or fractures.

[Zirconia Sintered Body]

The following describes the zirconia sintered body.

The zirconia sintered body is obtained by firing the zirconia pre-sintered body obtained in the manner described above.

The stabilizer content (the content of yttria and stabilizers other than yttria) specified for the zirconia pre-sintered body is also applicable to a zirconia sintered body of the present invention.

In a zirconia sintered body of the present invention, when the first translucency of the zirconia sintered body fabricated by firing at 1,550°C for 7 minutes with a rate of temperature increase of 350°C/min is compared with the second translucency of the zirconia sintered body fabricated by firing at 1,550°C for 120 minutes with a rate of temperature increase of 10°C/min, it is preferable that the first translucency be 85% or more, more preferably 86% or more, even more preferably 90% or more, particularly preferably 95% or more of the second translucency, most preferably essentially the same as the second translucency.

This allows a zirconia sintered body of the present invention to maintain high translucency comparable to that achieved by long sintering, even when sintered for a short period with a hold time of 7 minutes or less at 1,550°C.

A zirconia sintered body of the present invention may comprise a fluorescent agent. The fluorescent agent may be the same fluorescent agent used in the zirconia pre-sintered body.

In this specification, concerning the fluorescent agent, the phrase "relative to 100 mass% of zirconia contained in zirconia pre-sintered body" can be read as "relative to 100 mass% of zirconia contained in zirconia sintered body."

A zirconia sintered body of the present invention may comprise a colorant. The colorant may be the same colorant used in the zirconia pre-sintered body.

The colorant content in the zirconia sintered body is not particularly limited, and may be appropriately adjusted according to factors such as the type of colorant, and intended use of the zirconia sintered body. However, in view of considerations such as suitability in dental prosthesis applications, the colorant content is preferably 0.001 mass% or more, more preferably 0.005 mass% or more, even more preferably 0.01 mass% or more in terms of the oxide equivalent of the metal elements contained in the colorant, relative to 100 mass% of zirconia contained in the zirconia sintered body. The colorant content is preferably 5 mass% or less, more preferably 1 mass% or less, even more preferably 0.5 mass% or less in terms of the oxide equivalent of the metal elements contained in the colorant. The colorant content may be 0.1 mass% or less, or 0.05 mass% or less.

A zirconia sintered body of the present invention may comprise a translucency adjuster, in order to adjust the translucency of the zirconia sintered body. The translucency adjuster may be the same translucency adjuster used in the zirconia pre-sintered body.

The content of the translucency adjuster in the zirconia sintered body is not particularly limited, and may be appropriately adjusted according to factors such as the type of translucency adjuster, and intended use of the zirconia sintered body. However, in view of considerations such as suitability in dental prosthesis applications, the content of the translucency adjuster is preferably 0.1 mass% or less relative to 100 mass% of zirconia contained in the zirconia sintered body.

The relative density of the zirconia sintered body is preferably 99.0% or more, more preferably 99.2% or more, even more preferably 99.5% or more.

The relative density can be calculated as a ratio of the actual density, measured by the Archimedes method, with respect to the theoretical density.

A greater density in the zirconia sintered body leads to fewer internal voids and less scattering of light, and thereby improves translucency. From this perspective, the relative density is preferably 5.80 g/cm$^3$ or more, more preferably 5.82 g/cm$^3$ or more, even more preferably 5.87 g/cm$^3$ or more. Particularly preferably, the zirconia sintered body is essentially void free.

It is preferable for the zirconia sintered body to exhibit higher mechanical strength. For example, the mechanical strength is preferably 800 MPa or more, more preferably 820 MPa or more, even more preferably 840 MPa or more in terms of a biaxial flexural strength. The biaxial flexural strength can be measured in compliance with ISO 6872: 2015.

[Production Method of Zirconia Sintered Body]

A zirconia sintered body of the present invention can be produced by, for example, a method that fires the zirconia pre-sintered body.

Preferably, the method comprises the step of firing the zirconia pre-sintered body at more than 1,200°C and 1,700°C or less under ordinary pressure (hereinafter, also referred to as "firing step").

By employing such a method, a zirconia sintered body of the present invention can be easily produced that exhibits excellent translucency even after short sintering with a hold time of 2 minutes at the highest sintering temperature.

The production method of the present invention also enables easy production of a zirconia sintered body of the present invention that can maintain high translucency comparable to that achieved by long firing, even when sintered for a short period with a hold time of 7 minutes or less at 1,550°C.

When the sintered body is produced by firing a zirconia pre-sintered body of the present invention, it is preferable to employ highest sintering temperature conditions that maximize the translucency of the zirconia sintered body.

In view of considerations such as allowing easy production of the desired zirconia sintered body under ordinary pressure, the highest sintering temperature is preferably more than 1,200°C, more preferably 1,250°C or more, even more preferably 1,300°C or more. The highest sintering temperature is preferably 1,700°C or less, more preferably 1,650°C or less, even more preferably 1,600°C or less.

When the highest sintering temperature is at or above the foregoing lower limits and at or below the upper limits specified above, the sintering process can sufficiently proceed, easily yielding a dense sintered body. When the highest sintering temperature is at or below the foregoing upper limits, it is possible to inhibit deactivation of the fluorescent agent.

The highest sintering temperature is not particularly limited, as long as it falls within the foregoing temperature ranges, and is higher than the pre-sintering temperature (highest pre-sintering temperature) of the pre-sintering step. For example, the highest sintering temperature may be set to be at least 50°C, 100°C, 200°C, or 300°C higher than the highest pre-sintering temperature.

When producing the sintered body, the sintering time is not particularly limited, as long as the hold time at the highest sintering temperature is 7 minutes or less. However, in view of considerations such as enabling efficient and stable production of the desired zirconia sintered body with good productivity, the hold time at the highest sintering temperature is preferably 5 minutes or less, more preferably 4 minutes or less, even more preferably 3 minutes or less, particularly preferably 2 minutes or less.

The hold time is preferably 30 seconds or more, more preferably 45 seconds or more, even more preferably 1 minute or more.

In producing the zirconia sintered body using a zirconia pre-sintered body of the present invention, a shorter firing time can be employed for the fabrication of the sintered body without causing a decrease in the translucency of the zirconia sintered body prepared. Particularly, the hold time at the highest sintering temperature can be reduced to 7 minutes or less (for example, 2 minutes) for the fabrication of the sintered body. This can lead to enhanced production efficiency, and, when a zirconia pre-sintered body of the present invention is applied to dental products, the patient can experience less time-related stress because the time required from deciding the product dimensions and performing machining to making the dental product ready for treatment can be reduced. It is also possible to reduce the energy cost. When the hold time at the highest sintering temperature is at or below the foregoing upper limits, it is also possible to inhibit deactivation of the fluorescent agent.

Preferably, the rate of temperature increase and the rate of temperature decrease in the firing process are set so as to reduce the time required for the firing process. For example, the rate of temperature increase may be set so that the highest sintering temperature is reached in the shortest possible time, depending on the capabilities of the furnace. The rate of temperature increase to the highest sintering temperature may be, for example, 10°C/min or more, 50°C/min or more, 100°C/min or more, 120°C/min or more, 150°C/min or more, 200°C/min or more, 250°C/min or more, 300°C/min or more, or 350°C/min or more. Preferably, the rate of temperature decrease is set to a rate that does not cause defects, such as cracks, in the sintered body. For example, the sintered body may be allowed to cool at room temperature after the heating is finished.

In the present invention, a firing furnace can be used for pre-sintering and firing. The type of furnace is not particularly limited, and may be, for example, a furnace used in industry, such as an electric furnace or debinding furnace.

It is also possible to use a commercially available dental firing furnace (for example, Sintra CS manufactured by Shenpaz under this trade name).

A zirconia sintered body of the present invention can be produced even without a HIP process. However, the translucency and mechanical strength can further improve when a HIP process, as an optional process, follows the sintering performed under ordinary pressure.

In the following, the term "primary sintered body" is used to refer to a sintered body obtained through sintering at the highest sintering temperature (sintered body before HIP processing), whereas "HIP sintered body" is used to refer to a sintered body after HIP processing.

Known hot isostatic pressing (HIP) devices can be used for HIP processing.

The HIP pressure in the HIP process of the primary sintered body is not particularly limited. However, for advantages such as obtaining a dense sintered body with high mechanical strength, the HIP pressure is preferably 100 MPa or more, more preferably 125 MPa or more, even more preferably 130 MPa or more. The upper limit of HIP pressure is not particularly limited, and may be, for example, 400 MPa or less, 300 MPa or less, or 200 MPa or less.

The rate of temperature increase in the HIP process of the primary sintered body is not particularly limited, and is preferably 0.1°C/min or more, more preferably 0.2°C/min or more, even more preferably 0.5°C/min or more. The rate of temperature increase is preferably 50°C/min or less, more preferably 30°C/min or less, even more preferably 20°C/min or less. Productivity improves when the rate of temperature increase is at or above the foregoing lower limits.

The duration of HIP in the HIP process of the primary sintered body (the duration in which the highest pressure and temperature are maintained) is not particularly limited. However, for advantages such as obtaining a dense zirconia sintered body with high mechanical strength, the duration of the HIP process is preferably 5 minutes or more, more preferably 10 minutes or more, even more preferably 30 minutes or more. The duration of the HIP process is preferably 10 hours or less, more preferably 6 hours or less, even more preferably 3 hours or less.

In the method for producing a zirconia sintered body of the present invention, the pressure medium in the HIP process of the primary sintered body is not particularly limited. However, in view of a low impact on zirconia, the pressure medium may be at least one selected from the group consisting of oxygen, oxygen with 3% hydrogen, air, and various inert gases (for example, nitrogen, argon).

When conducting HIP processing of the primary sintered body in an oxygen-mixed gas atmosphere, the oxygen concentration may be, for example, more than 0% and 20% or less, though it is not particularly limited.

When using an oxygen-mixed gas, at least one inert gas (for example, such as nitrogen or argon) may be selected as a gas other than oxygen.

The HIP process may result in blackening due to oxygen deficiency when the HIP process is carried out in a reducing atmosphere such as by using an inert gas. In order to prevent such blackening, it is preferable to include a heat treatment step (or tempering as it is also called hereinbelow), performed at 1,650°C or less in either the atmosphere or an excess oxygen atmosphere, after the HIP process. In view of the efficiency of heat treatment, the heat treatment step is more preferably carried out in an excess oxygen atmosphere.

Here, excess oxygen atmosphere means an atmosphere where the oxygen concentration exceeds that of the atmosphere.

The excess oxygen atmosphere is not particularly limited, as long as the oxygen concentration is more than 21% and 100% or less, and can be appropriately selected within this range. For example, the oxygen concentration may be 100%. The HIP process may result in blackening due to oxygen deficiency when the HIP process is carried out in a reducing atmosphere such as by using an inert gas. In order to prevent such blackening, it is preferable to include a heat treatment step (or tempering as it is also called hereinbelow), performed at 1,650°C or less in either the atmosphere or an excess oxygen atmosphere, after the HIP process. In view of the efficiency of heat treatment, the heat treatment step is more preferably carried out in an excess oxygen atmosphere. Here, excess oxygen atmosphere means an atmosphere where the oxygen concentration exceeds that of the atmosphere. The excess oxygen atmosphere is not particularly limited, as long as the oxygen concentration is more than 21% and 100% or less, and can be appropriately selected within this range. For example, the oxygen concentration may be 100%.

A zirconia sintered body of the present invention may be a primary sintered body, a HIP sintered body, or a tempered sintered body with no particular restriction, provided that the present invention can exhibit its effects.

The heat treatment temperature in the atmosphere or an excess oxygen atmosphere may be appropriately modified according the aesthetics of the zirconia sintered body (for example, the shade of dental prostheses).

In a certain preferred embodiment, in view of the aesthetics of the zirconia sintered body, the heat treatment temperature in the atmosphere or an excess oxygen atmosphere is preferably 1,650°C or less, more preferably 1,600°C or less, even more preferably 1,550°C or less.

In another preferred embodiment, in view of the aesthetics of the zirconia sintered body, the heat treatment temperature in the atmosphere or an excess oxygen atmosphere is preferably 1,400°C or less, more preferably 1,300°C or less, even more preferably 1,200°C or less.

In either embodiment, the heat treatment temperature is preferably 500°C or more, more preferably 600°C or more, even more preferably 700°C or more.

A common dental zirconia furnace can be used for the tempering process. The dental zirconia furnace may be a commercially available product. Examples of commercially available products include Noritake KATANA® F-1, F-1N, F-2, F-2N (SK Medical Electronics Co., Ltd.).

The zirconia sintered body produced by firing a zirconia pre-sintered body of the present invention can be suitably used as a dental product.

Examples of such dental products include copings, frameworks, crowns, crown bridges, abutments, implants, implant screws, implant fixtures, implant bridges, implant burs, brackets, denture bases, inlays, onlays, orthodontic wires, and laminate veneers.

Using a zirconia pre-sintered body of the present invention for components such as implant screws and implant fixtures can reduce gingival discoloration associated with the use of metallic materials, thereby enhancing aesthetics.

Additionally, a suitable producing method can be chosen depending on the intended application. For example, a dental product can be obtained by firing a zirconia pre-sintered body of the present invention after it has been machined. The use of a CAD/CAM system is preferred for the machining process.

The CAD/CAM system is not particularly limited, and known devices may be used. Examples of such devices include known CAD/CAM systems such as the KATANA® CAD/CAM system manufactured by Kuraray Noritake Dental Inc.

A certain preferred embodiment (X-1) is, for example, a zirconia sintered body comprising zirconia, and a stabilizer capable of preventing a phase transformation of zirconia, wherein at least one zirconia crystal grain contained in the zirconia sintered body has a region with a concentration gradient where the stabilizer concentration (content) within the same crystal grain decreases from the central side of the crystal grain toward its outer crystal grain boundary.

In the zirconia sintered body according to this embodiment (X-1), there may be regions that exhibit different patterns of increase or decrease in the stabilizer content due to conditions such as variations (concentration unevenness) in the content of the stabilizer, provided that at least one zirconia crystal grain has a region having the aforementioned concentration gradient.

The stabilizer in the zirconia sintered body according to embodiment (X-1) may be the same yttria or the same non-yttria stabilizer capable of preventing a phase transformation of zirconia that may be used in a zirconia pre-sintered body according to the present invention.

In view of even superior translucency, the zirconia sintered body according to embodiment (X-1) is preferably one in which the stabilizer capable of preventing a phase transformation of zirconia is yttria.

The zirconia sintered body according to embodiment (X-1) is preferably a zirconia sintered body in which the concentration gradient is 0.1 to 3 mol% relative to the total moles of zirconia and the stabilizer.

In view of superior translucency, the concentration gradient (the difference in content between the central side and the crystal grain boundary) is preferably 0.15 mol% or more, more preferably 0.2 mol% or more, even more preferably 0.3 mol% or more, particularly preferably 0.5 mol% or more. In view of superior translucency, the concentration gradient is preferably 2.8 mol% or less, more preferably 2.5 mol% or less, even more preferably 2.2 mol% or less, particularly preferably 2.0 mol% or less.

The method for evaluating the concentration gradient is as described in the EXAMPLES below.

The following describes the zirconia sintered body according to embodiment (X-1) based on a preferred example of a zirconia sintered body where the stabilizer capable of preventing a phase transformation of zirconia is yttria.

The zirconia sintered body according to embodiment (X-1) exhibits excellent translucency even when the hold time at the highest sintering temperature is 2 minutes in firing the zirconia pre-sintered body comprising $Y_4Zr_3O_{12}$ as a crystal system.

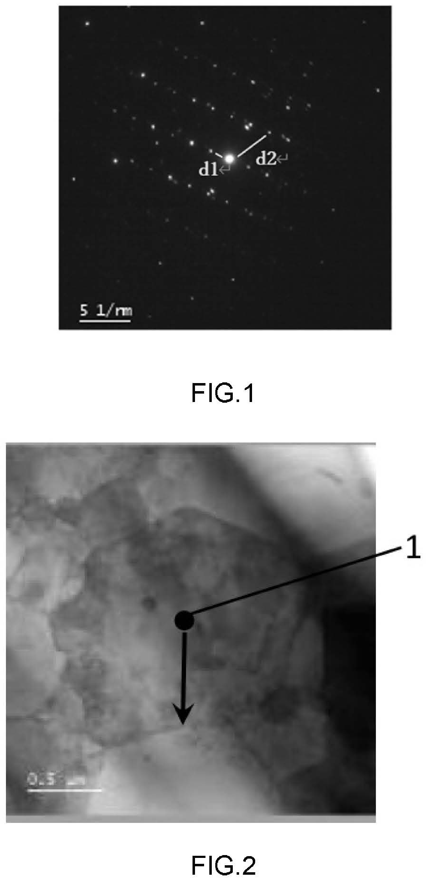

The zirconia sintered body possessing the aforementioned capabilities has a region with a concentration gradient where the yttria content decreases from the central side (for example, a central portion 1) toward the outer crystal grain boundary, as indicated by the direction of the arrow in FIG. 2 under a transmission electron microscope (TEM).

The central side may refer to any central portion within the same crystal grain, and may be, for example, the centroid (geometric center). The centroid can be determined using known analytical software (for example, Image-Pro Plus, image analysis software manufactured by Hakuto Co., Ltd. under this trade name). The centroid can be found from the contour of the crystal grain.

It has been known from conventional techniques that, in the final stage of sintering, the yttria concentration within the same crystal grain exhibits a gradient with decreasing concentration of yttria from the outer crystal grain boundary of the crystal grain toward the center when observed under a transmission electron microscope (TEM). This is a completely opposite pattern from that observed in the zirconia sintered body according to embodiment (X-1).

The method of production of the zirconia sintered body according to embodiment (X-1) is not particularly limited, as long as the zirconia sintered body has the crystal grain structure described above. In a preferred method, a zirconia pre-sintered body comprising $Y_4Zr_3O_{12}$ as a crystal system is prepared through a pulverization process performed under applied energy in excess of that conventionally employed while both zirconia and yttria are present. The zirconia pre-sintered body can then be fired for a short period (for example, with a hold time of 2 minutes at the highest sintering temperature) to produce the zirconia sintered body according to embodiment (X-1).

In the zirconia sintered body according to embodiment (X-1), it is believed that short firing (for example, with a hold time of 2 minutes at the highest sintering temperature) allows yttria to have a concentration gradient in at least one of the zirconia crystal grains.

In the zirconia sintered body according to embodiment (X-1), the yttria concentration gradient is thought to provide low-yttria areas at the peripheral regions (crystal grain boundaries) of zirconia crystal grains, including cubic crystals, which are typically highly translucent but mechanically weak. It is believed that these areas of low yttria concentration, acting as a mechanism that improves toughness (stress-induced phase transformation), provide excellent mechanical strength and translucency in the zirconia sintered body, even when it is produced by short firing (for example, with a hold time of 2 minutes at the highest sintering temperature).

The yttria content in zirconia crystal grains can be measured by, for example, scanning transmission electron microscopy with energy dispersive X-ray spectroscopy (STEM-EDS).

A more preferred embodiment (X-1-1) is, for example, a zirconia sintered body comprising zirconia, and a stabilizer capable of preventing a phase transformation of zirconia, obtained by firing a zirconia pre-sintered body that comprises zirconia and yttria components, along with $Y_4Zr_3O_{12}$ contained as a crystal system, wherein at least one zirconia crystal grain contained in the zirconia sintered body has a region with a concentration gradient where the stabilizer concentration within the same crystal grain decreases from the central side of the crystal grain toward its outer crystal grain boundary.

Another certain preferred embodiment (X-2) is, for example, a zirconia sintered body comprising zirconia, and a stabilizer capable of preventing a phase transformation of zirconia, wherein the zirconia sintered body comprises:

a zirconia crystal grain (L) in which the stabilizer content is 4 to 6 mol% relative to the total moles of zirconia and the stabilizer; and
a zirconia crystal grain (S) in which the stabilizer content is 1 to 3 mol% relative to the total moles of zirconia and the stabilizer.

The number of zirconia crystal grains (L) observed in a 5 $\mu$m $\times$ 5 $\mu$m region under a TEM is preferably three or more, more preferably five or more, even more preferably seven or more, particularly preferably ten or more. The number of zirconia crystal grains (S) observed in a 5 $\mu$m $\times$ 5 $\mu$m region under a TEM is preferably five or more, more preferably ten or more, even more preferably fifteen or more, particularly preferably twenty or more.

The zirconia sintered body according to embodiment (X-2) may comprise, without particular restriction, zirconia crystal grains in which the stabilizer content is more than 3 mol% and less than 4 mol% relative to the total moles of zirconia and the stabilizer.

The stabilizer in the zirconia sintered body according to embodiment (X-2) may be the same yttria or the same non-yttria stabilizer capable of preventing a phase transformation of zirconia that may be used in a zirconia pre-sintered body according to the present invention.

In view of even superior translucency, the zirconia sintered body according to embodiment (X-2) is preferably one in which the stabilizer capable of preventing a phase transformation of zirconia is yttria.

In view of even superior translucency and mechanical strength, the zirconia sintered body according to embodiment (X-2) is preferably a zirconia sintered body in which the zirconia crystal grains (L) have an average crystal grain diameter in a range of 0.5 to 2.0 $\mu$m, and the zirconia crystal grains (S) have an average crystal grain diameter in a range of 0.1 to 0.3 $\mu$m.

In view of even superior translucency and mechanical strength, the average crystal grain diameter of the zirconia crystal grains (L) in the zirconia sintered body according to embodiment (X-2) is preferably 0.5 $\mu$m or more, more preferably 0.6 $\mu$m or more, even more preferably 0.7 $\mu$m or more, particularly preferably 0.8 $\mu$m or more.

In view of even superior translucency and mechanical strength, the average crystal grain diameter of the zirconia crystal grains (L) is preferably 2.0 $\mu$m or less, more preferably 1.5 $\mu$m or less, even more preferably 1.3 $\mu$m or less, particularly preferably 1.2 $\mu$m or less.

In view of even superior translucency and mechanical strength, the average crystal grain diameter of the zirconia crystal grains (S) in the zirconia sintered body according to embodiment (X-2) is preferably 0.10 $\mu$m or more, more preferably 0.12 $\mu$m or more, even more preferably 0.15 $\mu$m or more, particularly preferably 0.18 $\mu$m or more.

In view of even superior translucency and mechanical strength, the average crystal grain diameter of the zirconia crystal grains (S) is preferably 0.3 $\mu$m or less, more preferably 0.28 $\mu$m or less, even more preferably 0.25 $\mu$m or less, particularly preferably 0.22 $\mu$m or less.

The method for measuring the average crystal grain diameter of zirconia crystal grains (L) and zirconia crystal grains (S) is as described in the EXAMPLES below.

The following describes the zirconia sintered body according to embodiment (X-2) based on a preferred example of a zirconia sintered body where the stabilizer capable of preventing a phase transformation of zirconia is yttria.

The method of production of the zirconia sintered body according to embodiment (X-2) is not particularly limited, as long as the zirconia sintered body has the crystal grain structure described above. In a preferred method, a zirconia pre-sintered body comprising $Y_4Zr_3O_{12}$ as a crystal system is prepared through a pulverization process performed under applied energy in excess of that conventionally employed while both zirconia and yttria are present. The zirconia pre-sintered body can then be fired for a short period (for example, with a hold time of 2 minutes at the highest sintering temperature) to produce the zirconia sintered body according to embodiment (X-2).

In the zirconia sintered body according to embodiment (X-2), it is believed that short firing (for example, with a hold time of 2 minutes at the highest sintering temperature) yields the zirconia sintered body comprising zirconia crystal grains (L) and zirconia crystal grains (S).

In the zirconia sintered body according to embodiment (X-2), the presence of zirconia crystal grains (L) and zirconia crystal grains (S) with the stabilizer content (preferably, yttria content) and the average crystal grain diameters specified above is thought to enable a reduction in light interference in the visible wavelength range while ensuring mechanical strength with fine crystal grains. It is believed that this provides excellent mechanical strength and translucency in the zirconia sintered body, even when it is produced by short firing (for example, with a hold time of 2 minutes at the highest sintering temperature).

The zirconia sintered body according to embodiment (X-2) exhibits excellent translucency even when the hold time at the highest sintering temperature is 2 minutes in firing the zirconia pre-sintered body comprising $Y_4Zr_3O_{12}$ as a crystal system.

In the zirconia sintered body according to embodiment (X-2), the presence of a high yttria content region with zirconia crystal grains (L) with a high yttria content of 4 to 6 mol%, and a low yttria content region with zirconia crystal grains (S) with a yttria content of 1 to 3 mol% in the zirconia sintered body is thought to provide a crystal structure combining high-strength tetragonal crystals and high-transparency cubic crystals, achieving excellent strength and translucency as a sintered body.

A more preferred embodiment (X-2-1) is, for example, a zirconia sintered body comprising zirconia, and a stabilizer capable of preventing a phase transformation of zirconia, obtained by firing a zirconia pre-sintered body that comprises zirconia and yttria components, along with $Y_4Zr_3O_{12}$ contained as a crystal system, wherein the zirconia sintered body comprises:

a zirconia crystal grain (L) in which the stabilizer concentration is 4 to 6 mol% relative to the total moles of zirconia and the stabilizer; and
a zirconia crystal grain (S) in which the stabilizer concentration is 1 to 3 mol% relative to the total moles of zirconia and the stabilizer.

In view of even superior translucency and mechanical strength, the zirconia sintered body according to embodiment (X-2) is preferably a zirconia sintered body that comprises:

a zirconia crystal grain (Lx) in which the stabilizer concentration is 5 to 6 mol% relative to the total moles of zirconia and the stabilizer; and
a zirconia crystal grain (Sx) in which the stabilizer concentration is 1 to 2 mol% relative to the total moles of zirconia and the stabilizer.

In view of even superior translucency and mechanical strength, the zirconia sintered body comprising a zirconia crystal grain (Lx) and a zirconia crystal grain (Sx) is more preferably one in which the zirconia crystal grain (Lx) has an average crystal grain diameter within the range of the average crystal grain diameters of zirconia crystal grains (L), and the zirconia crystal grain (Sx) has an average crystal grain diameter within the range of the average crystal grain diameters of zirconia crystal grains (S).

Yet another certain preferred embodiment (X-3) is, for example, a zirconia sintered body that comprises zirconia, and a stabilizer capable of preventing a phase transformation of zirconia, wherein at least one zirconia crystal grain contained in the zirconia sintered body has a region with a concentration gradient where the stabilizer concentration within the same crystal grain decreases from the central side of the crystal grain toward its outer crystal grain boundary, and the zirconia crystal grains comprise a zirconia crystal grain (L) in which the stabilizer content is 4 to 6 mol% relative to the total moles of zirconia and the stabilizer, and a zirconia crystal grain (S) in which the stabilizer content is 1 to 3 mol% relative to the total moles of zirconia and the stabilizer.

The concentration gradient of the zirconia crystal grains in the zirconia sintered body according to embodiment (X-3) is as described in the embodiment (X-1) above.

The zirconia crystal grain (L) and zirconia crystal grain (S) in the zirconia sintered body according to embodiment (X-3) are as described in the embodiment (X-2) above.

In embodiment (X-3), in view of even superior translucency and mechanical strength, the zirconia crystal grains with a region having the concentration gradient are zirconia crystal grains (L).

The zirconia sintered body according to embodiment (X-3) is preferably one in which ten arbitrarily selected zirconia crystal grains (L) in a TEM image (one field) under a TEM contains preferably five or more, more preferably six or more, even more preferably seven or more zirconia crystal grains (L) having the concentration gradient.

If a single filed cannot provide ten zirconia crystal grains (L), additional fields may be added to select a total of ten zirconia crystal grains (L).

Likewise, in this specification, if the required number of grains cannot be observed in one field when selecting a specific number of crystal grains from one field (for example, 10 or 20), an additional field or fields may be used to select the required total number of grains (for example, 10 or 20).

A more preferred embodiment (X-3-1) is, for example, a zirconia sintered body comprising zirconia and yttria, obtained by firing a zirconia pre-sintered body that comprises zirconia and yttria components, along with $Y_4Zr_3O_{12}$ contained as a crystal system, wherein at least one zirconia crystal grain contained in the zirconia sintered body has a region with a concentration gradient where the yttria concentration (content) within the same crystal grain decreases from the central side of the crystal grain toward its outer crystal grain boundary, and the zirconia crystal grains comprise a zirconia crystal grain (L) in which the yttria content is 4 to 6 mol% relative to the total moles of zirconia and yttria, and a zirconia crystal grain (S) in which the yttria content is 1 to 3 mol% relative to the total moles of zirconia and yttria.

More preferably, another more preferred embodiment (X-3-2) is a zirconia sintered body that comprises a zirconia crystal grain (Lx) in which the stabilizer concentration is 5 to 6 mol% relative to the total moles of zirconia and the stabilizer, and a zirconia crystal grain (Sx) in which the stabilizer concentration is 1 to 2 mol% relative to the total moles of zirconia and the stabilizer, and in which the zirconia crystal grains with a region having the concentration gradient of stabilizer are zirconia crystal grains (Lx).

Even more preferably, in view of even superior translucency and mechanical strength, yet another more preferred embodiment (X-3-3) is a zirconia sintered body that comprises a zirconia crystal grain (Lx) and a zirconia crystal grain (Sx), and in which the zirconia crystal grain (Lx) has an average crystal grain diameter within the range of the average crystal grain diameters of zirconia crystal grains (L), and the zirconia crystal grain (Sx) has an average crystal grain diameter within the range of the average crystal grain diameters of zirconia crystal grains (S).

The zirconia sintered body according to embodiment (X-3) may comprise, without particular restriction, zirconia crystal grains in which the stabilizer content is more than 3 mol% and less than 4 mol% relative to the total moles of zirconia and the stabilizer.

The present invention encompasses embodiments combining all or part of the foregoing features in various ways within the technical idea of the present invention, provided that the present invention can exhibit its effects.

For example, in preferred embodiments (X-1) to (X-3), all or part of the features may be combined in various ways within the scope described in this specification.

Specifically, the present invention encompasses embodiments in which the zirconia sintered bodies according to preferred embodiments (X-1) to (X-3) have a biaxial flexural strength of 800 MPa or more.

EXAMPLES

The present invention is described below in greater detail through Examples. It is to be noted, however, that the present invention is in no way limited by the following Examples, and various modifications may be made by a person with ordinary skill in the art within the technical idea of the present invention.

[Example 1]

A commercially available zirconia powder ($Y_2O_3$: 0 mol%) and a commercially available yttria powder were charged into water. The mixture, along with a pulverization medium made of zirconia (diameter: 2 mm), was placed in the vessel of a ball mill, and pulverized for 144 hours with the ball mill to obtain a slurry (average particle diameter: 0.2 $\mu$m or less).

Subsequently, an organic binder was added into the slurry. After mixing and stirring, the slurry was dried and granulated with a spray dryer to obtain a powder.

The powder was poured into a columnar mold, uniaxially pressed at 200 MPa pressure to obtain a molded body.

The molded body was placed in an electric furnace, heated from room temperature at 10°C/min, and retained at 500°C for 2 hours to debind the organic components. The resulting material was then heated at 10°C/min, held at 955°C (highest pre-sintering temperature) for 2 hours, and allowed to cool at -10°C/min to obtain a zirconia pre-sintered body.

[Examples 2 to 11 and Comparative Examples 1 to 4]

The zirconia pre-sintered body was produced following the same method described in Example 1, except that the conditions indicated in Table 1 were applied. Examples 2 to 11 and Comparative Examples 1 to 4 used the same raw material for yttria as that used in Example 1.

In Example 4, pulverization was carried out with a bead mill using a zirconia pulverization medium of 0.1 mm diameter, instead of using a ball mill. The pulverization time was modified as indicated in Table 1.

In Example 11, pulverization was carried out in the same manner as in Example 1, using a ball mill. This was followed by pulverization with a bead mill using a zirconia pulverization medium of 0.1 mm diameter. The pulverization time was modified as indicated in Table 1 for each pulverization process.

In Table 1, the 1.5Y zirconia ($Y_2O_3$: 1.5 mol%) was prepared as follows.

A commercially available zirconia powder ($Y_2O_3$: 0 mol%) and a commercially available yttria powder were charged into water to yield a yttria content of 1.5 mol% relative to the total mol% of zirconia and yttria. The mixture, along with a pulverization medium made of zirconia (diameter: 2 mm), was placed in the vessel of a ball mill, and pulverized for 48 hours with the ball mill to obtain a slurry (average particle diameter: 0.2 μm or less). Subsequently, the slurry was dried and granulated with a spray dryer to obtain a powder. The powder was then heated, and held at 1,000°C for 100 hours to obtain the raw material.

In Table 1, the 40Y zirconia ($Y_2O_3$: 40 mol%) was prepared as follows.

A commercially available zirconia powder ($Y_2O_3$: 0 mol%) and a commercially available yttria powder were charged into water to yield a yttria content of 40 mol% relative to the total mol% of zirconia and yttria. The mixture, along with a pulverization medium made of zirconia (diameter: 2 mm), was placed in the vessel of a ball mill, and pulverized for 48 hours with the ball mill to obtain a slurry (average particle diameter: 0.2 μm or less). The slurry was dried and granulated with a spray dryer to obtain a powder. The powder was then heated, and held at 1,000°C for 100 hours to obtain the raw material.

[Table 1]

| | Yttria content (mol%) | Raw materials used | Pulverization method | Pulverization time (h) | Pre-sintering temperature (°C) |
|---|---|---|---|---|---|
| Ex. 1 | 5.5 | Monoclinic zirconia + Yttria | Ball mill | 144 | 955 |
| Ex. 2 | 3 | Monoclinic zirconia + Yttria | Ball mill | 144 | 955 |
| Ex. 3 | 4.5 | Monoclinic zirconia + Yttria | Ball mill | 144 | 955 |
| Ex. 4 | 4.5 | Monoclinic zirconia + Yttria | Bead mill | 1 | 875 |
| Ex. 5 | 8 | Monoclinic zirconia + Yttria | Ball mill | 144 | 955 |
| Ex. 6 | 5.5 | Monoclinic zirconia + Yttria | Ball mill | 48 | 955 |
| Ex. 7 | 5.5 | 1.5Y zirconia + Yttria | Ball mill | 144 | 955 |
| Ex. 8 | 5.5 | Monoclinic zirconia + 40Y zirconia | Ball mill | 144 | 955 |
| Ex. 9 | 5.5 | 1.5Y zirconia + 40Y zirconia | Ball mill | 144 | 955 |
| Ex. 10 | 5.5 | Commercially available product TZ-3YS + 40Y zirconia | Ball mill | 144 | 955 |
| Ex. 11 | 4.5 | Monoclinic zirconia + Yttria | Ball mill + Bead mill | 20 (Ball mill) + 0.5 (Bead mill) | 950 |
| Com. Ex. 1 | 3 | Commercially available product TZ-3YS | No pulverization | 0 | 955 |
| Com. Ex. 2 | 4 | Commercially available product Zpex 4 | No pulverization | 0 | 955 |
| Com. Ex. 3 | 5.2 | Commercially available product Zpex Smile | No pulverization | 0 | 955 |
| Com. Ex. 4 | 5.5 | Commercially available product TZ-3YS + Yttria | Ball mill | 20 | 955 |

In the table, the yttria content refers to the mole percentage of yttria relative to the total number of moles of zirconia and yttria (mol%).

In the table, the commercially available products in Example 10 and in Comparative Examples 1 to 4 are the products manufactured by Tosoh Corporation.

<Evaluation for Presence or Absence of $Y_4Zr_3O_{12}$ in Zirconia Pre-Sintered Body>

The zirconia pre-sintered body obtained in each Example and Comparative Example was evaluated for the presence or absence of $Y_4Zr_3O_{12}$, using a TEM.

Specifically, the pre-sintered body was polished to a thickness of 30 $\mu$m, and its surface was processed using a precision ion polishing system (Model 691 PIPS manufactured by Gatan (US) under this trade name). From the prepared sample, an electron diffraction image was obtained, using an atomic-resolution analytical electron microscope (JEM-ARM200F ACCELARM manufactured by JEOL Ltd. under this trade name) with a camera length of 1.0 cm and an accelerating voltage of 200 kV.

First, as shown in FIG. 1, measurements were taken from the diffraction image to determine the distances $d_1$ and $d_2$ between diffraction spots (corresponding to the interplanar spacings) and the angle between them (corresponding to the interplanar angle $\varphi$).

Next, using formulae (1-3) and (1-4), which calculate the interplanar spacing and interplanar angle of a hexagonal crystal system by substituting the lattice constants of $Y_4Zr_3O_{12}$ (a = 9.738Å, c = 9.115Å), it was examined whether there is any combination of Miller indices $(h_1, k_1, l_1)$ and $(h_2, k_2, l_2)$ corresponding to the measured interplanar spacings $(d_1, d_2)$ and interplanar angle $(\varphi)$. The lattice constants of $Y_4Zr_3O_{12}$ were taken from "MAGNETIC SUSCEPTIBILITY OF M4Zr3O12 AND M4Hf3O12 (M - RARE-EARTH ELEMENT)" Inorg. Mater., 1991, 27, pp.1495-1497, Red'ko V.P., Lopato L.M.

$$1/d^2 = 4/3((h^2 + hk + k^2)/a^2) + l^2/c^2 \hspace{2cm} (1\text{-}3)$$

$$\cos\varphi = (h_1h_2 + k_1k_2 + 1/2(h_1k_2 + h_2k_1) + (3a^2/4c^2)l_1l_2)/((h_1{}^2 + k_1{}^2 + h_1k_1 + (3a^2/4c^2)l_1{}^2)(h_2{}^2 + k_2{}^2 + h_2k_2 + (3a^2/4c^2)l_2{}^2))^{1/2} \hspace{0.5cm} (1\text{-}4)$$

When evaluated following this procedure, the presence of $Y_4Zr_3O_{12}$ was confirmed in the zirconia pre-sintered bodies of Examples 1 to 11, whereas it was absent in the zirconia pre-sintered bodies of Comparative Examples 1 to 4.

<Evaluation of Abundance of Crystal Systems in Zirconia Pre-Sintered Body>

The abundance $f_d$ of $Y_4Zr_3O_{12}$, along with the tetragonal fraction $f_t$, cubic fraction $f_c$, and monoclinic fraction $f_m$ in the zirconia pre-sintered body obtained in each Example and Comparative Example were determined by analyzing the crystal systems of the zirconia pre-sintered body.

Specifically, the integrated intensities of the respective peaks (peak integrated intensities I) were determined through X-ray diffraction measurement conducted under the conditions below, using Automated Horizontal Multipurpose X-Ray Diffractometer (SmartLab, manufactured by Rigaku Corporation) and X-Ray Analysis Integrated Software (SmartLab Studio II, manufactured by Rigaku Corporation).

    X-ray source: Cu K$\alpha$ ($\lambda$ = 1.54186 Å)
    Goniometer length: 300 mm
    Optical system: focusing technique
    Detector: high-speed one-dimensional X-ray detector (D/teX Ultra 250)
    Monochromatization: K$\beta$ filter
    Tube voltage: 40 kV
    Tube current: 30 mA
    Scan axis: 2$\theta$/$\theta$
    Scan speed: 0.2°/min
    Sampling step: 0.01°

The abundance $f_d$ (%) of $Y_4Zr_3O_{12}$, and the monoclinic fraction $f_m$ (%) were calculated from the integrated intensities of the respective peaks, using the formulae (1-1) and (1-2) above. The results are presented in Table 2.

Because the main peaks of $Y_4Zr_3O_{12}$, the tetragonal zirconia, and the cubic zirconia occur at similar positions in the calculation of the abundance $f_d$ (%) of $Y_4Zr_3O_{12}$, the peaks were separated when these crystal systems were shown to coexist. Specifically, these crystal systems were examined first to confirm their presence or absence.

For $Y_4Zr_3O_{12}$, its presence or absence was confirmed by the TEM evaluation described earlier.

The presence or absence of the tetragonal zirconia was confirmed based on the subpeaks near 34.6° and 35.4°.

For the cubic zirconia, its presence or absence was confirmed based on the subpeak near 35.0°.

This was followed by peak separation based on the crystal systems detected. For example, when all three crystal systems were present, the overlapping peaks of the three crystal systems appearing near 30°-$Y_4Zr_3O_{12}$ with the peak top at 29.6°, the tetragonal crystal with the peak top at 30.3°, and the cubic crystal with the peak top at 30.0°-were separated by adjusting the width at half maximum and the peak intensity at the respective peak top positions so that each peak takes the form of an approximation curve of the Gaussian function. The integrated intensities $I_d$, $I_t$, and $I_c$ were then calculated for the respective crystal systems. When two crystal systems were present, these were separated into two peaks, and the integrated intensity was calculated for each peak.

<Evaluation of Crystal Grains of Zirconia Sintered Body>

The zirconia crystal grains in the zirconia sintered body were identified, and the yttria content of the zirconia crystal grains, the average crystal grain diameters of zirconia crystal grains (L) and zirconia crystal grains (S), and the concentration gradient of yttria in the zirconia crystal grains were measured, using the following procedures.

<Method for Identifying Zirconia Crystal Grains>

Zirconia crystal grains (a zirconia concentration of 80 mass% or more) were identified as follows.

Under bright-field TEM (at 20,000× magnification), particles including an observation point (a 5 × 5 nm observation area) with a zirconia concentration of 80 mass% or more as measured by scanning transmission electron microscopy with energy dispersive X-ray spectroscopy (STEM-EDS) and visually appearing nearly uniform in brightness were regarded as individual zirconia crystal grains.

<Yttria Content of Zirconia Crystal Grains>

In the zirconia crystal grains identified as described above, the yttria content was measured using STEM-EDS. Here, a square observation area was defined, with at least three of its vertices inscribed within the grain boundary of the crystal grain, and its square shape maximized to fit within the crystal grain.

The zirconia sintered body of Example 1, obtained by firing using the method described below in <Evaluation of Translucency of Zirconia Sintered Body (Measurement 1 of ΔL*(W-B))> (short firing with a 2-minute retention at 1,580°C), contained at least one hundred zirconia crystal grains with a yttria content of 4 to 6 mol%, and at least one hundred zirconia crystal grains with a yttria content of 1 to 3 mol% (counted across 10 fields of view).

The zirconia sintered body of Example 1 also contained at least one hundred zirconia crystal grains with a yttria content of 5 to 6 mol%, and at least one hundred zirconia crystal grains with a yttria content of 1 to 2 mol% (counted across 10 fields of view).

In contrast, in the zirconia sintered body of Comparative Example 3, only zirconia crystal grains within the 4 to 6 mol% yttria were present, and it was not possible to detect zirconia crystal grains falling within the 1 to 3 mol% yttria.

<Method for Measuring Average Crystal Grain Diameter of Zirconia Crystal Grains (L) and Zirconia Crystal Grains (S)>

Zirconia crystal grains with a yttria content of 4 to 6 mol% were designated as zirconia crystal grains (L), and those with a yttria content of 1 to 3 mol% were designated as zirconia crystal grains (S). The equivalent circular diameter (area-equivalent circular diameter) of each crystal grain, identified using the previously described method for identifying zirconia crystal grains based on TEM observation, was then taken as its particle diameter, and the arithmetic mean of the equivalent circular diameters of one hundred arbitrarily selected crystal grains was calculated as the average crystal grain diameter of the zirconia crystal grains.

More specifically, ten crystal grains were arbitrarily selected per field, and the arithmetic mean of the equivalent circular diameters of these crystal grains was calculated across ten fields to determine the average crystal grain diameter of the zirconia crystal grains.

In the zirconia sintered body of Example 1, the average crystal grain diameter was 1.0 $\mu$m for zirconia crystal grains (L), and 0.2 $\mu$m for zirconia crystal grains (S).

As an alternative approach, zirconia crystal grains with a yttria content of 5 to 6 mol% may be designated as zirconia crystal grains (Lx), and those with a yttria content of 1 to 2 mol% as zirconia crystal grains (Sx). Following this approach, the zirconia sintered body of Example 1 had an average crystal grain diameter of 1.2 $\mu$m for zirconia crystal grains (Lx), and 0.2 $\mu$m for zirconia crystal grains (Sx).

<Measurement of Concentration Gradient of Yttria in Zirconia Crystal Grains>

In the zirconia crystal grains identified through TEM observation as previously described, the yttria content was measured at the central portion and the crystal grain boundary, using STEM-EDS (with an observation area of $5 \times 5$ nm). Here, the measurement at the central portion was taken at the centroid of the crystal grain, whereas the content at the crystal grain boundary was measured within a region on the boundary side corresponding to 1/10 of the distance from the crystal grain boundary to the centroid. In the zirconia sintered body of Example 1, the yttria content was 5.9 mol% at the central portion (centroid), and 4.2 mol% at the crystal grain boundary in an arbitrarily selected zirconia crystal grain (L). In ten zirconia crystal grains (L) arbitrarily selected from multiple fields, the yttria content at the crystal grain boundary was found to be at least 0.15 mol% lower than at the central portion (centroid) in nine of these zirconia crystal grains.

In contrast, in the zirconia sintered body of Comparative Example 3, the yttria content was the same at the central portion (centroid) and the crystal grain boundary of the zirconia crystal grains, or higher at the crystal grain boundary than at the central portion (centroid).

The centroid of the crystal grain was determined from its contour using image analysis software (Image-Pro Plus manufactured by Hakuto Co., Ltd. under this trade name).

<Evaluation of Translucency of Zirconia Sintered Body (Measurement 1 of $\Delta L^*$(W-B))>

The zirconia pre-sintered body obtained in each Example and Comparative Example was subjected to short firing involving a 2-minute retention time at 1,580°C with a rate of temperature increase of 350°C/min, using a dental furnace (Sintra CS manufactured by Shenpaz under this trade name).

The resulting zirconia sintered body was polished into a flat plate sample with a thickness of 1.0 mm and used for translucency measurement. The translucency measurement was carried out using a spectrophotometer (Crystaleye manufactured by Olympus Corporation under this trade name) in 7-band measurement mode with an LED light source.

Following the procedure described in <Evaluation of Translucency of Zirconia Sintered Body (Measurement 2 of $\Delta L^*$(W-B))> below, the lightness (LW*) was found from chromaticity measurement against a white background. The same sample was also used to find the lightness (LB*) from chromaticity measurement against a black background, using the same measurement instrument in the same measurement mode with the same light source. The difference between these values ($\Delta L^* = (LW^*) - (LB^*)$) was then determined as the translucency ($\Delta L^*$(W-B)) (arithmetic mean value for n = 3).

A translucency $\Delta L^*$(W-B) of 13 or greater was considered acceptable. The results are presented in Table 2 below.

<Evaluation of Translucency of Zirconia Sintered Body (Measurement 2 of $\Delta L^*$(W-B))>

The pre-sintered body obtained in each Example and Comparative Example was fired to prepare a zirconia sintered body (first sintered body). The pre-sintered body was fired at a highest sintering temperature of 1,550°C, with a rate of temperature increase of 350°C/min and a hold time of 7 minutes at the highest sintering temperature (7-minute firing).

Following this, a pre-sintered body prepared using the same procedure was fired to prepare another zirconia sintered body (second sintered body). The pre-sintered body was fired at a highest sintering temperature of 1,550°C, with a rate of temperature increase of 10°C/min and a hold time of 120 minutes at the highest sintering temperature (120-minute firing).

The rate of temperature decrease was set at 200°C/min for 7-minute firing, and 10°C/min for 120-minute firing.

These two zirconia sintered bodies were each polished into a flat plate sample with a thickness of 1.0 mm and used for translucency measurement. The translucency measurement was carried out using a spectrophotometer (Crystaleye manufactured by Olympus Corporation under this trade name) in 7-band measurement mode with an LED light source.

Specifically, the translucency of the zirconia sintered body, prepared as a flat plate sample, was measured by finding the lightness (LW*) and lightness (LB*) from chromaticity measurement against a white background and a black background, respectively, using the same sample. The measurements were carried out using the same measurement instrument in the same measurement mode with the same light source. The difference between these values ($\Delta L^* = (LW^*) - (LB^*)$) was then determined as the translucency ($\Delta L^*$(W-B)) (arithmetic mean value for n = 3). The L* value is an L* value in the chromaticity (color space) of the L*a*b* color system (JIS Z 8781-4:2013).

The first sintered body, prepared by firing at 1,550°C for 7 minutes, and the second sintered body, prepared by firing at 1,550°C for 120 minutes, were measured to determine the first translucency $\Delta L_1^*$(W-B) and the second translucency $\Delta L_2^*$(W-B), respectively. The ratio of $\Delta L_1^*$(W-B) to $\Delta L_2^*$(W-B) ($\Delta L_1^*$(W-B)/$\Delta L_2^*$(W-B)) was then calculated as a rate of change in translucency.

As a rate of change in translucency, a value of 0.85 or greater (85% or more) was considered acceptable. The results are presented in Table 2 below.

<Strength Evaluation of Zirconia Sintered Body>

The zirconia sintered body of Example 1, obtained by firing following the procedure described in <Evaluation of Translucency of Zirconia Sintered Body (Measurement 1 of ΔL*(W-B))> above (short firing with a 2-minute retention time at 1,580°C), was measured for biaxial flexural strength directly after being polished into a flat plate sample with a thickness of 1.0 mm. The measurement was conducted using a universal testing machine AGS-X (manufactured by Shimadzu Corporation) with the crosshead speed set at 1.0 mm/min, following ISO6872:2015 (n = 5). The arithmetic mean was calculated to be 911 MPa.

In contrast, the commercially available product of Comparative Example 3 yielded a biaxial flexural strength of 600 MPa after the same measurement and calculation following Example 1.

[Table 2]

| | Pre-sintered body | | Sintered body | |
|---|---|---|---|---|
| | fd (%) | fm (%) | Measurement 1: ΔL*(W-B) (Sample thickness: 1.0 mm) | Measurement 2: Translucency difference (7-minute retention vs. 120-minute retention) |
| Ex. 1 | 8 | 40 | 16 | 89% |
| Ex. 2 | 6.1 | 44 | 14 | 86% |
| Ex. 3 | 7 | 41 | 15 | 87% |
| Ex. 4 | 11.3 | 44 | 15 | 92% |
| Ex. 5 | 8.9 | 29 | 16 | 88% |
| Ex. 6 | 4.8 | 47 | 13 | 87% |
| Ex. 7 | 4.2 | 39 | 14 | 88% |
| Ex. 8 | 15.2 | 48 | 14 | 87% |
| Ex. 9 | 14.2 | 35 | 13 | 88% |
| Ex. 10 | 12.5 | 0 | 13 | 87% |
| Ex. 11 | 3.5 | 44 | 13 | 86% |
| Com. Ex. 1 | 0 | 0 | 5 | 38% |
| Com. Ex. 2 | 0 | 0 | 10 | 71% |
| Com. Ex. 3 | 0 | 0 | 3 | 20% |
| Com. Ex. 4 | 0 | 0 | 12 | 80% |

These results confirmed that the zirconia pre-sintered body of the present invention can yield a zirconia sintered body that exhibits superior translucency even with a 2-minute hold time at the highest sintering temperature.

By using the zirconia pre-sintered body of the present invention, the highest sintering temperature can be set below 1,600°C. For example, with a highest sintering temperature of 1,550°C, the zirconia sintered body sintered for a short period with a hold time of 7 minutes or less at 1,550°C was able to maintain high translucency comparable to that achieved by long sintering.

In Comparative Examples, a 2-minute hold time at the highest sintering temperature did not allow sintering to progress sufficiently to attain the target translucency.

It was also demonstrated that the zirconia sintered bodies with excellent translucency, produced by firing the pre-sintered bodies with a hold time of 2 minutes at the highest sintering temperature, show zirconia crystal grain characteristics that differ from those seen in sintered bodies produced by conventional techniques.

INDUSTRIAL APPLICABILITY

A zirconia pre-sintered body of the present invention is useful for the production of dental products used in dental treatments at dental clinics.

**Claims**

1. A zirconia pre-sintered body comprising zirconia and yttria, along with $Y_4Zr_3O_{12}$ as a crystal system.

2. The zirconia pre-sintered body according to claim 1, wherein the abundance $f_d$ (%) of $Y_4Zr_3O_{12}$ is 2.5% or more as calculated by the following formula (1-1),

$$f_d\ (\%) = I_d\ /\ (I_m + I_t + I_c + I_y + I_d) \times 100 \qquad (1\text{-}1)$$

wherein $f_d$ represents the abundance (%) of $Y_4Zr_3O_{12}$, $I_m$ represents the integrated intensity of the peak near $2\theta = 28.2°$, where the peak top of the main peak of the monoclinic zirconia appears in XRD measurement, $I_t$ represents the integrated intensity of the peak near $2\theta = 30.3°$, where the peak top of the main peak of the tetragonal zirconia appears in XRD measurement, $I_c$ represents the integrated intensity of the peak near $2\theta = 30.0°$, where the peak top of the main peak of the cubic zirconia appears in XRD measurement, $I_d$ represents the integrated intensity of the peak near $2\theta = 29.6°$, where the peak top of the main peak of $Y_4Zr_3O_{12}$ appears in XRD measurement, and $I_y$ represents the integrated intensity of the peak near $2\theta = 29.2°$, where the peak top of the main peak of undissolved yttria appears in XRD measurement, and
given that the main peaks of $Y_4Zr_3O_{12}$, the tetragonal zirconia, and the cubic zirconia occur at similar positions, the integrated intensity of each main peak is calculated through peak separation.

3. The zirconia pre-sintered body according to claim 1 or 2, wherein the zirconia comprises monoclinic zirconia, and the monoclinic fraction $f_m$ (%) is less than 55% as calculated by the following formula (1-2),

$$f_m\ (\%) = I_m\ /\ (I_m + I_t + I_c + I_y + I_d) \times 100 \qquad (1\text{-}2),$$

wherein $f_m$ represents the monoclinic fraction (%), $I_m$ represents the integrated intensity of the peak near $2\theta = 28.2°$, where the peak top of the main peak of the monoclinic zirconia appears in XRD measurement, $I_t$ represents the integrated intensity of the peak near $2\theta = 30.3°$, where the peak top of the main peak of the tetragonal zirconia appears in XRD measurement, $I_c$ represents the integrated intensity of the peak near $2\theta = 30.0°$, where the peak top of the main peak of the cubic zirconia appears in XRD measurement, $I_d$ represents the integrated intensity of the peak near $2\theta = 29.6°$, where the peak top of the main peak of $Y_4Zr_3O_{12}$ appears in XRD measurement, and $I_y$ represents the integrated intensity of the peak near $2\theta = 29.2°$, where the peak top of the main peak of undissolved yttria appears in XRD measurement, and
given that the main peaks of $Y_4Zr_3O_{12}$, the tetragonal zirconia, and the cubic zirconia occur at similar positions, the integrated intensity of each main peak is calculated through peak separation.

4. The zirconia pre-sintered body according to claim 1 or 2, wherein the yttria content is 2.5 mol% or more and 10 mol% or less relative to the total moles of the zirconia and the yttria.

5. A zirconia pre-sintered body comprising zirconia and yttria,
wherein, in comparison, the first translucency of a first sintered body fabricated by firing the zirconia pre-sintered body at 1,550°C for 7 minutes with a rate of temperature increase of 350°C/min is 85% or more of the second translucency of a second sintered body fabricated by firing the zirconia pre-sintered body at 1,550°C for 120 minutes with a rate of temperature increase of 10°C/min.

6. A method for producing a zirconia pre-sintered body of claim 1 or 2, comprising the steps of:

   producing a zirconia composition that comprises zirconia particles and yttria particles;
   pulverizing the zirconia composition;
   molding the zirconia composition after pulverization to obtain a molded body; and
   pre-sintering the molded body,
   wherein the pulverization step involves (i) pulverization for a period of 10 minutes or more using a pulverization medium of less than 1 mm in diameter, or (ii) pulverization for a period of more than 40 hours using a pulverization medium of 1 mm or more in diameter.

7. The method for producing a zirconia pre-sintered body according to claim 6, wherein the pre-sintering step involves a pre-sintering temperature of 600 to 1,300°C.

8. A zirconia sintered body comprising zirconia, and a stabilizer capable of preventing a phase transformation of zirconia, wherein at least one zirconia crystal grain contained in the zirconia sintered body has a region with a concentration gradient where the stabilizer concentration within the same crystal grain decreases from the central side of the crystal grain toward its outer crystal grain boundary.

9. The zirconia sintered body according to claim 8, wherein the concentration gradient is 0.1 to 3 mol% relative to the total moles of the zirconia and the stabilizer.

10. The zirconia sintered body according to claim 8 or 9, wherein the stabilizer capable of preventing a phase transformation of zirconia is yttria.

11. A zirconia sintered body comprising zirconia, and a stabilizer capable of preventing a phase transformation of zirconia, wherein the zirconia sintered body comprises:

a zirconia crystal grain (L) in which the stabilizer content is 4 to 6 mol% relative to the total moles of the zirconia and the stabilizer; and
a zirconia crystal grain (S) in which the stabilizer content is 1 to 3 mol% relative to the total moles of the zirconia and the stabilizer.

12. The zirconia sintered body according to claim 11, wherein:

the zirconia crystal grain (L) has an average crystal grain diameter in a range of 0.5 to 2.0 $\mu$m, and
the zirconia crystal grain (S) has an average crystal grain diameter in a range of 0.1 to 0.3 $\mu$m.

13. The zirconia sintered body according to claim 11 or 12, wherein the stabilizer capable of preventing a phase transformation of zirconia is yttria.

FIG.1

FIG.2

# EP 4 782 420 A1

## INTERNATIONAL SEARCH REPORT

International application No.

**PCT/JP2024/033773**

### A. CLASSIFICATION OF SUBJECT MATTER

*C04B 35/488*(2006.01)i; *A61C 5/77*(2017.01)i; *A61C 13/083*(2006.01)i; *A61K 6/818*(2020.01)i; *A61K 6/822*(2020.01)i
FI: C04B35/488; A61C5/77; A61C13/083; A61K6/818; A61K6/822

According to International Patent Classification (IPC) or to both national classification and IPC

### B. FIELDS SEARCHED

Minimum documentation searched (classification system followed by classification symbols)

C04B35/488; A61C5/77; A61C13/083; A61K6/818; A61K6/822

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2023/120371 A1 (KCM CORP.) 29 June 2023 (2023-06-29) paragraphs [0081]-[0101] | 1-13 |
| A | JP 6-056527 A (KUROSAKI REFRACT CO., LTD.) 01 March 1994 (1994-03-01) paragraphs [0034]-[0041] | 1-13 |
| A | JP 2018-052806 A (TOSOH CORP.) 05 April 2018 (2018-04-05) entire text, all drawings | 1-13 |
| A | JP 2020-105050 A (KURARAY NORITAKE DENTAL INC.) 09 July 2020 (2020-07-09) entire text, all drawings | 1-13 |
| P, A | WO 2023/238861 A1 (KCM CORP.) 14 December 2023 (2023-12-14) entire text, all drawings | 1-13 |

☐ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **05 November 2024** | **19 November 2024** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

## INTERNATIONAL SEARCH REPORT
### Information on patent family members

International application No.

**PCT/JP2024/033773**

| Patent document cited in search report | | | Publication date (day/month/year) | Patent family member(s) | Publication date (day/month/year) |
|---|---|---|---|---|---|
| WO | 2023/120371 | A1 | 29 June 2023 | (Family: none) | |
| JP | 6-056527 | A | 01 March 1994 | (Family: none) | |
| JP | 2018-052806 | A | 05 April 2018 | (Family: none) | |
| JP | 2020-105050 | A | 09 July 2020 | (Family: none) | |
| WO | 2023/238861 | A1 | 14 December 2023 | (Family: none) | |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018056330 A

**Non-patent literature cited in the description**

- **RED'KO V.P.** ; **LOPATO L.M**. MAGNETIC SUSCEPTIBILITY OF M4Zr3O12 AND M4Hf3O12 (M - RARE-EARTH ELEMENT). *Inorg. Mater*, 1991, vol. 27, 1495-1497